# EUROPÄISCHE PATENTSCHRIFT

(11) **EP 0 906 292 B1**
(45) Veröffentlichungstag und Bekanntmachung des Hinweises auf die Patenterteilung: **09.07.2003**
(21) Anmeldenummer: 97920729.7
(22) Anmeldetag: 21.04.1997
(51) Int. Cl.: C07D 249/12, C07D 401/06, C07D 405/06, C07F 7/08, A01N 43/653, A01N 55/10

(54) **TRIAZOLYL-MERCAPTIDE UND IHRE VERWENDUNG ALS MIKROBIZIDE**
TRIAZOLYL MERCAPTIDES AND THEIR USE AS MICROBICIDES
TRIAZOLYL-MERCAPTIDES ET LEUR UTILISATION COMME MICROBICIDES

(30) Priorität: 30.04.1996 DE 19617282
(43) Veröffentlichungstag der Anmeldung: 07.04.1999
(73) Patentinhaber: Bayer CropScience AG, 40789 Monheim (DE)
(72) Erfinder: JAUTELAT, Manfred, D-51399 Burscheid (DE); DUTZMANN, Stefan, D-40764 Langenfeld (DE); STENZEL, Klaus, D-40595 Düsseldorf (DE)
(86) Internationale Anmeldenummer: EP9701996
(87) Internationale Veröffentlichungsnummer: WO97041107

(56) Entgegenhaltungen:
- EP-A- 0 251 086
- EP-A- 0 297 345
- WO-A-87/06430
- WO-A-96/16048
- WO-A-96/38423
- WO-A-96/38424
- WO-A-96/38440
- WO-A-96/39395
- WO-A-96/41798
- WO-A-96/41804
- WO-A-97/05119
- WO-A-97/06151
- WO-A-97/06152
- DE-A- 19 520 095

## Beschreibung

Die vorliegende Erfindung betrifft neue Triazolyl-mercaptide, ein Verfahren zu deren Herstellung und deren Verwendung als Mikrobizide.

Es ist bereits bekannt geworden, daß zahlreiche Triazolyl-Derivate fungizide Eigenschaften besitzen (vgl. EP-A 0 015 756, EP-A 0 040 345, EP-A 0 052 424, EP-A 0 061 835, EP-A 0 297 345, EP-A 0 094 564, EP-A 0 196 038, EP-A 0 267 778, EP-A 0 378 953, EP-A 0 068 813, EP-A 0 044 605, EP-A 0 069 442, EP-A 0 055 833, EP-A 0 301 393, DE-A 2 324 010, DE-A 2 737 489, DE-A 2 551 560, EP-A 0 065 485, DE-A 2 735 872, EP-A 0 234 242, DE-A 2 201 063, EP-A 0 145 294 und DE-A 3 721 786). Die Wirksamkeit dieser Stoffe ist gut, läßt aber bei niedrigen Aufwandmengen in manchen Fällen zu wünschen übrig

Aus der EP - A 0 251 086 und der WO 87- 06 430 sind fungizid wirksame Mercapto - Derivate von Silicium - Azolen und auch intermediär als Zwischenprodukte auftretende Lithium - Mercaptide von Azolen dieses Typs bekannt. Entsprechende Azol - Derivate, die kein Silicium enthalten, werden aber nicht erwähnt.

Aus der im Verhältnis zur vorliegenden Patentanmeldung vorgängigen, aber nicht vorveröffentlichten WO 96- 16 048 sind Metallsalz - Komplexe von bestimmten Mercapto - Triazol - Derivaten bekannt. Dabei handelt es sich um Substanzen, die durch Addition eines als Lewis - Säure wirkenden Metallsalzes an den basisch reagierenden Azolring entstehen. Verbindungen, in denen das Wasserstoffatom der Mercapto - Gruppe durch ein Kation ersetzt ist, werden jedoch nicht offenbart.

Es wurden nun neue Triazolyl-mercaptide der Formel 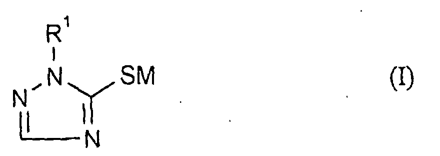 in welcher
- M: für ein Natrium- oder Kalium-Kation, ein Äquivalent eines Magnesium-, Calcium-, Kupfer-, Zink-, Eisen- oder Nickel-Kations oder für ein Ammonium-Kation der Formel 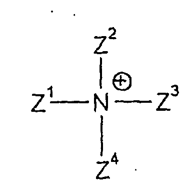 steht, in welcher
- Z¹: für Wasserstoff oder geradkettiges oder verzweigtes Alkyl mit 1 bis 8 Kohlenstoffatomen steht,
- Z²: für Wasserstoff oder geradkettiges oder verzweigtes Alkyl mit 1 bis 8 Kohlenstoffatomen steht,
- Z³: für Wasserstoff, geradkettiges oder verzweigtes Alkyl mit 1 bis 8 Kohlenstoffatomen, Benzyl oder Phenyl steht und
- Z⁴: für geradkettiges oder verzweigtes Alkyl mit 1 bis 18 Kohlenstoffatomen, Benzyl oder Phenyl steht,
und
- R¹: für einen Rest der Formel 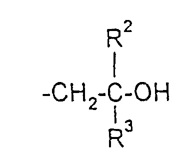 steht, in welcher
- R²: für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für geradkettiges oder verzweigtes Alkenyl mit 2 bis 6 Kohlenstoffatomen steht, wobei jeder dieser Reste einfach bis dreifach, gleichartig oder verschieden substituiert sein kann durch Halogen, Alkoxy mit 1 bis 4 Kohlenstoffatomen und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohienstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aralkenyl mit 6 bis 10 Kohlenstoffatomen im Arylteil und 2 bis 4 Kohlenstoffatomen im Alkenylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4
Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1-bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoff-. atomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano, und
- R³: für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für geradkettiges oder verzweigtes Alkenyl mit 2 bis 6 Kohlenstoffatomen steht, wobei jeder dieser Reste einfach bis dreifach, gleichartig oder verschieden substituiert sein kann durch Halogen, Alkoxy mit 1 bis 4 Kohlenstoffatomen und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aralkenyl mit 6 bis 10 Kohlenstoffatomen im Arylteil und 2 bis 4 Kohlenstoffatomen im Alkenylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano gefunden.

Zahlreiche der erfindungsgemäßen Stoffe enthalten eines oder mehrere asymmetrisch substituierte Kohlenstoffatome. Sie können daher in optischen Isomerenformen anfallen. Die vorliegende Erfindung betrifft sowohl die einzelnen Isomeren als auch deren Gemische.

Weiterhin wurde gefunden, daß man Triazolyl-mercaptide der Formel (I) erhält, wenn man Mercapto-triazole der Formel 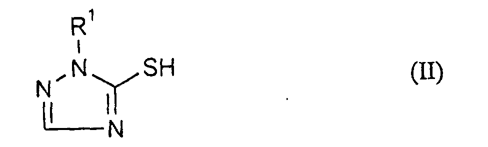 in welcher
- R¹: die oben angegebenen Bedeutungen hat,
entweder
a) mit Hydroxiden der Formel

   M¹-OH (III)

   in welcher
   - M¹: für Natrium- oder Kalium-Kation, ein Äquivalent eines Magnesium- oder Calcium-Kations oder für ein Ammonium-Kation der Formel 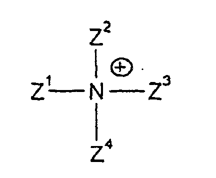 in welcher
   Z¹, Z², Z³ und Z⁴ die oben angegebenen Bedeutungen haben,
   in Gegenwart eines Verdünnungsmittels umsetzt,
   oder
b) mit Aminen der Formel 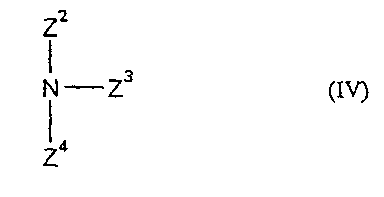 in welcher
   Z², Z³ und Z⁴ die oben angegebenen Bedeutungen haben,
   in Gegenwart eines Verdünnungsmittels umsetzt,
   oder
c) mit Acetaten der Formel

   CH₃-COO M² (V)

   in welcher
   - M²: für ein Äquivalent eines Kupfer-, Zink-, Eisen- oder Nickel-Kations steht,
in Gegenwart eines Verdünnungsmittels umsetzt.

Schließlich wurde gefunden, daß die neuen Triazolyl-mercaptide der Formel (I) sehr gute mikrobizide Eigenschaften aufweisen und sich sowohl im Pflanzenschutz als auch im Materialschutz zur Bekämpfung unerwünschter Mikroorganismen verwenden lassen.

Überraschenderweise besitzen die erfindungsgemäßen Stoffe eine bessere fungizide Wirksamkeit, insbesondere fungizide Wirksamkeit, als die konstitutionell ähnlichsten Verbindungen gleicher Wirkungsrichtung.

Die erfindungsgemäßen Triazolyl-mercaptide sind durch die Formel (I) allgemein definiert.
- M: steht bevorzugt für ein Natrium- oder Kalium-Kation, ein Äquivalent eines Magnesium-, Calcium-, Kupfer-, Zink-, Eisen- oder Nickel-Kations oder für ein Ammonium-Kation der Formel 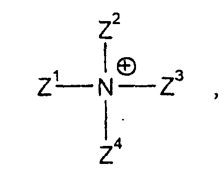 in welcher
Z¹ bevorzugt für Wasserstoff oder geradkettiges oder verzweigtes Alkyl mit 1 bis 6 Kohlenstoffatomen steht,
Z² bevorzugt für Wasserstoff oder geradkettiges oder verzweigtes Alkyl mit 1 bis 6 Kohlenstoffatomen steht,
Z³ bevorzugt für Wasserstoff, geradkettiges oder verzweigtes Alkyl mit 1 bis 6 Kohlenstoffatomen, Benzyl oder Phenyl steht und
Z⁴ bevorzugt für geradkettiges oder verzweigtes Alkyl mit 1 bis 18 Kohlenstoffatomen, Benzyl oder Phenyl steht.
- R¹ steht: bevorzugt für einen Rest der Formel 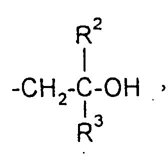 in welcher
R² bevorzugt für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei jeder dieser Reste einfach bis dreifach; gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl, und
R³ bevorzugt für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclo-. propyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl.

Die bei der Herstellung der erfindungsgemäßen Stoffe als Ausgangssubstanzen benötigten Mercapto-triazole können in der "Mercapto"-Form der Formel 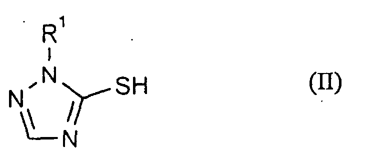 oder in der tautomeren "Thiono"-Form der Formel 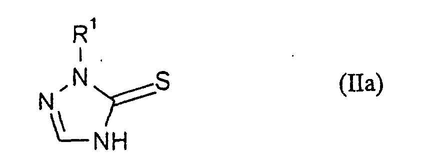 vorliegen. Es ist deshalb nicht auszuschließen, daß sich die erfindungsgemäßen Stoffe ganz oder teilweise von der "Thiono"-Form der Formel (IIa) ableiten. Das bedeutet, daß die erfindungsgemäßen Stoffe entweder als Substanzen der Formel 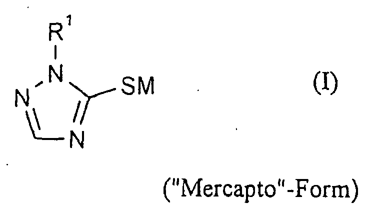 oder der Formel 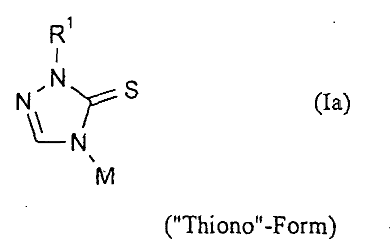 vorliegen oder eine mesomere Struktur der Formel 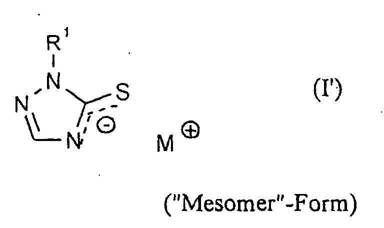 aufweisen. Der Einfachheit halber wird jeweils nur die "Mercapto"-Form angegeben.

Als Beispiele für erfindungsgemäße Stoffe seien die in der folgenden Tabelle aufgeführten Triazolyl-mercaptide genannt.

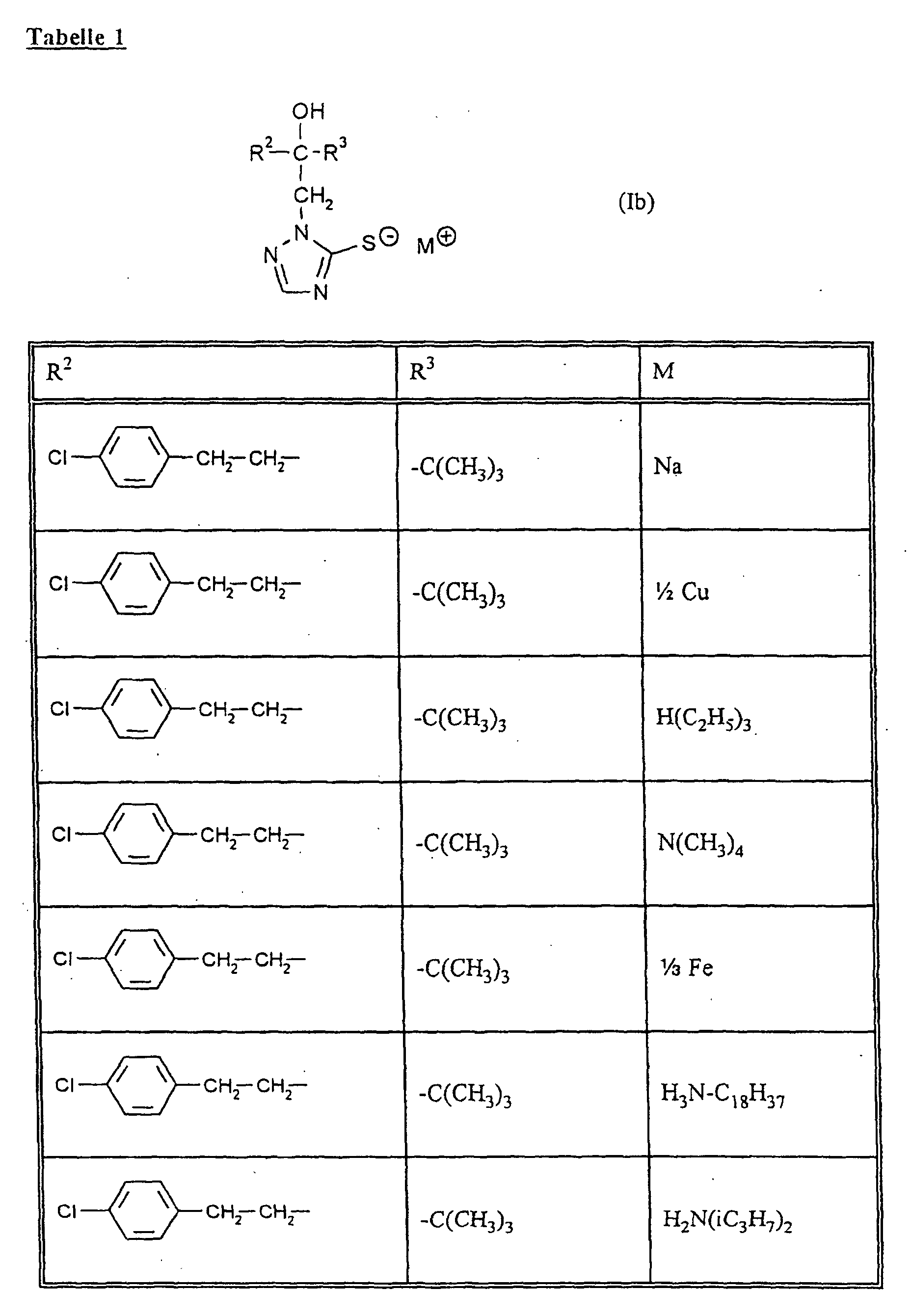

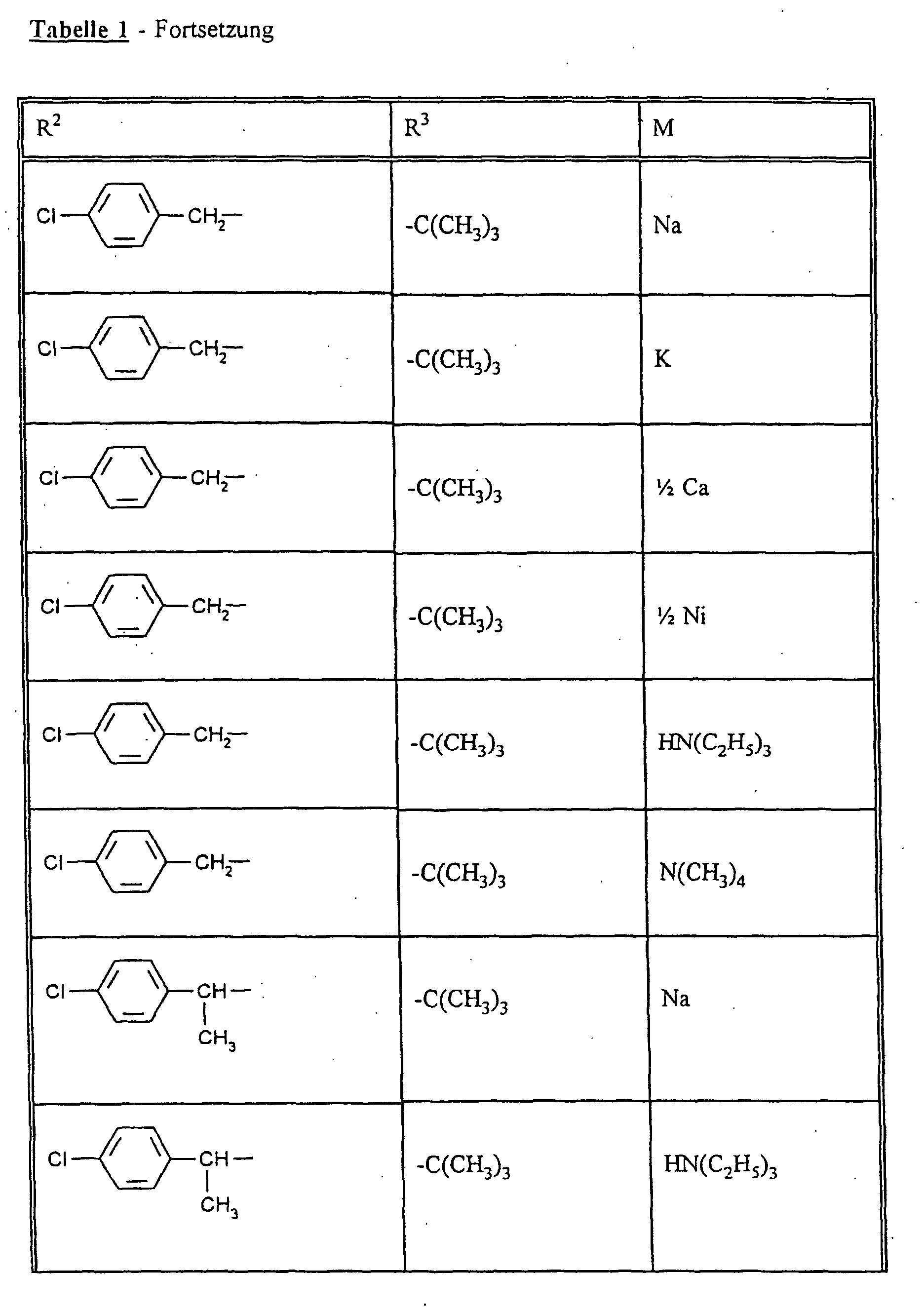

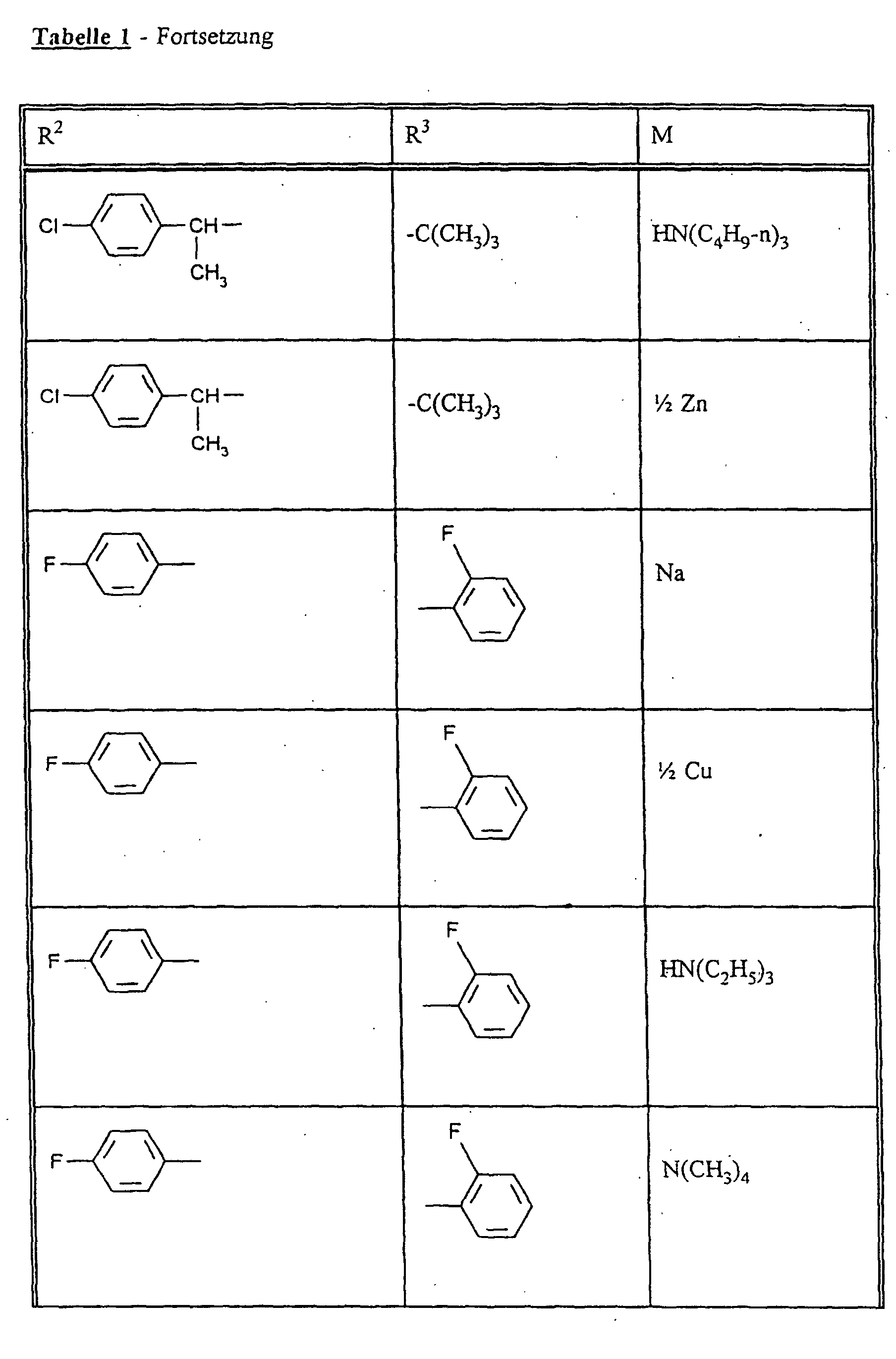

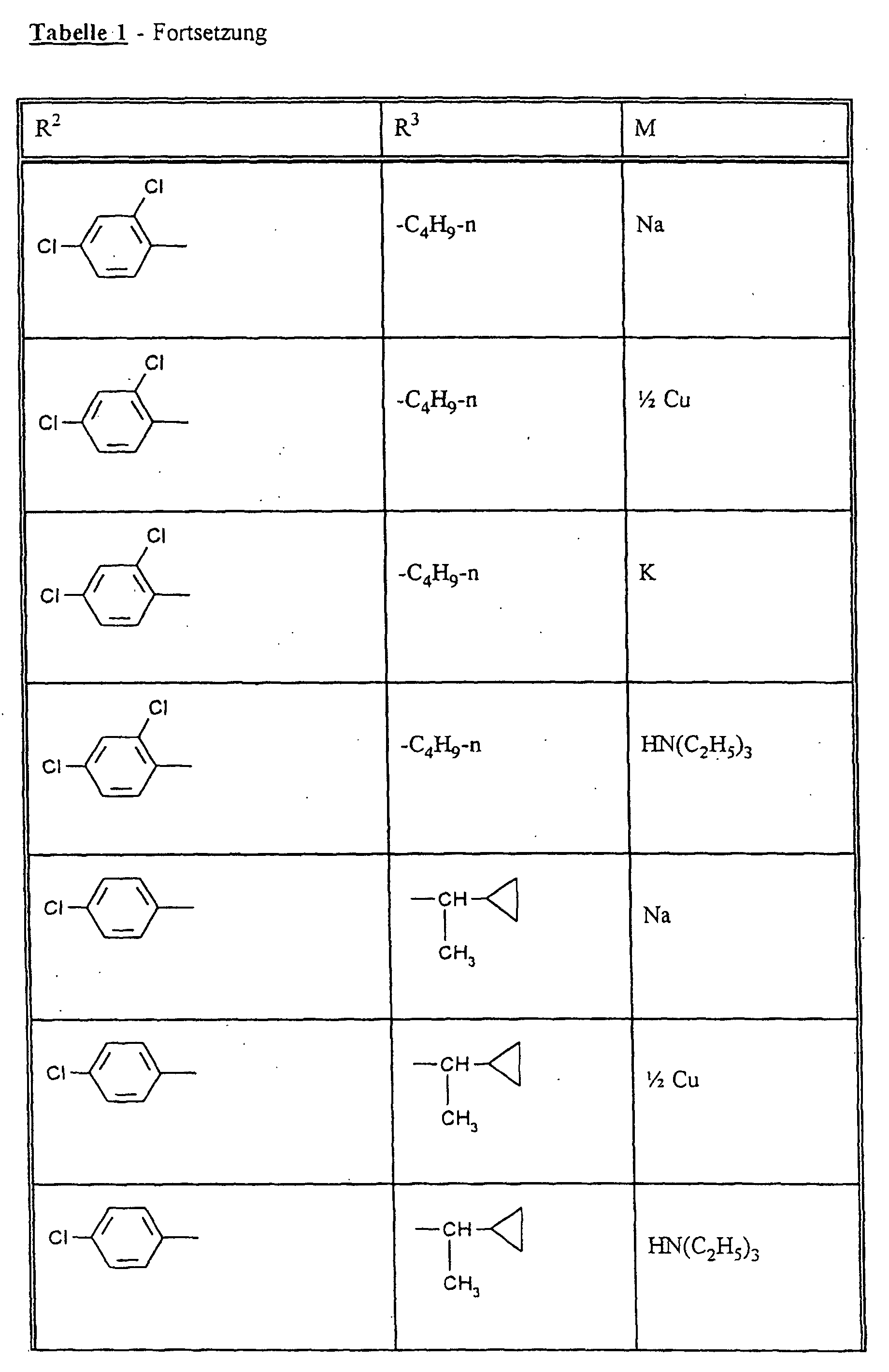

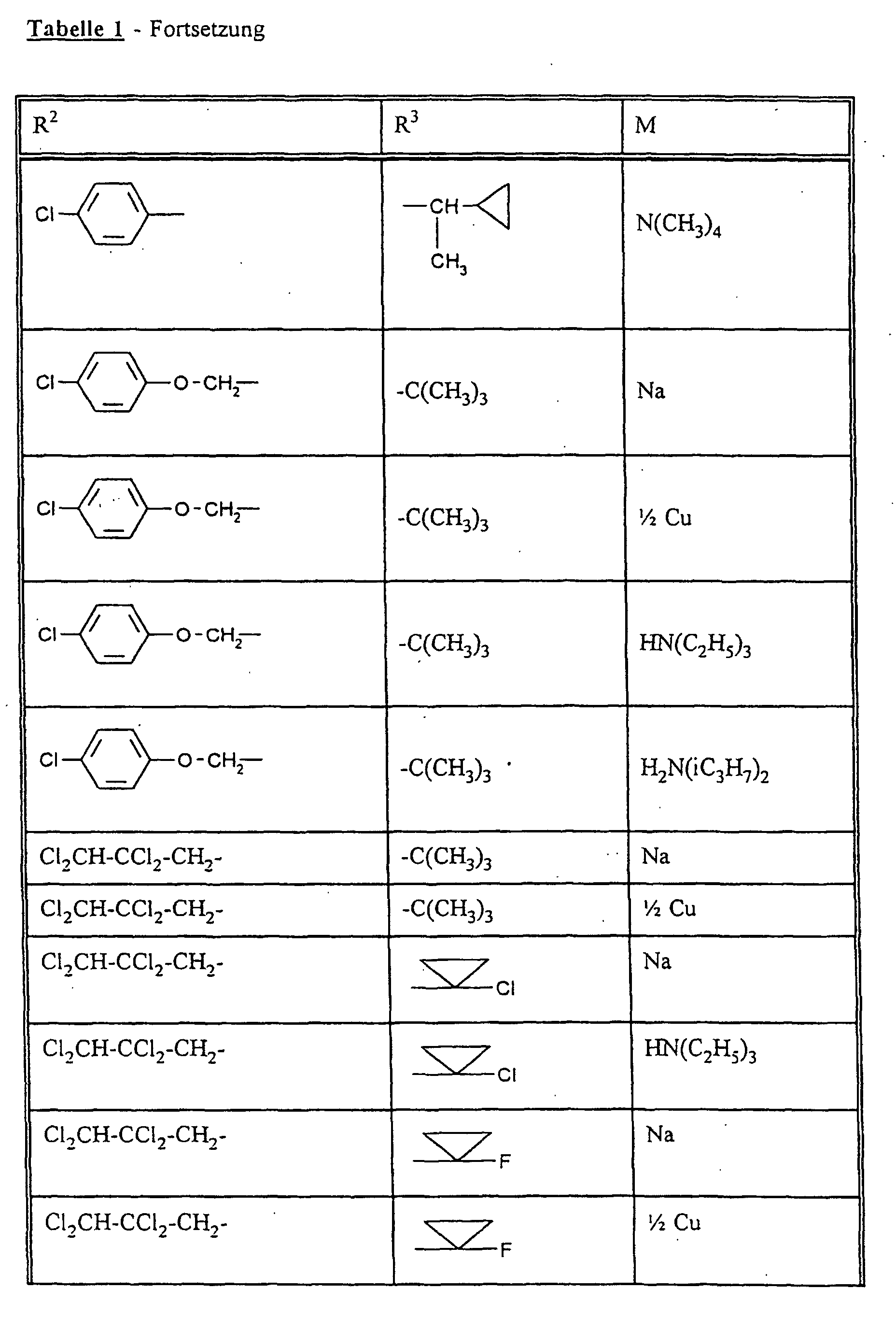

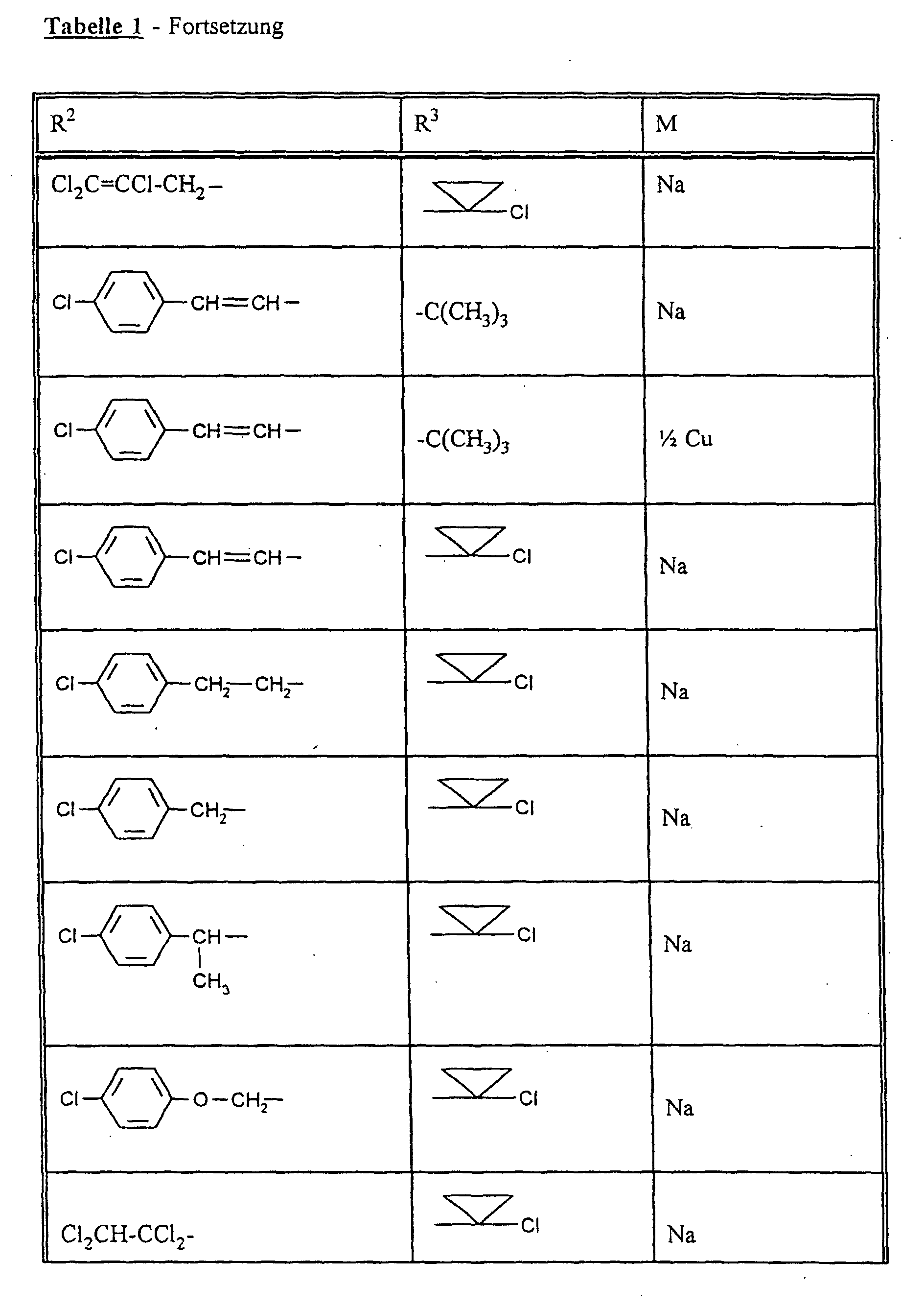

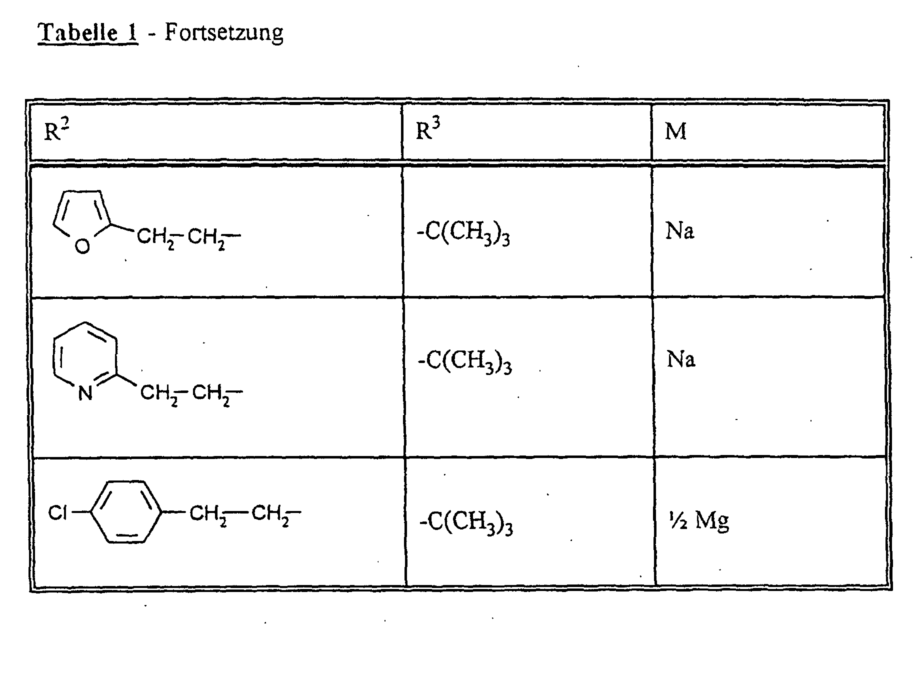

Verwendet man 2-(1-Chlor-cyclopropyl)-1-(2-chlorphenyl)-3-(5-mercapto-1,2,4-triazol-1-yl)-propan-2-ol als Ausgangsstoff und Natriumhydroxid als Reaktionskomponente, so kann der Verlauf des erfindungsgemäßen Verfahrens (Variante a) durch das folgende Formelschema veranschaulicht werden.

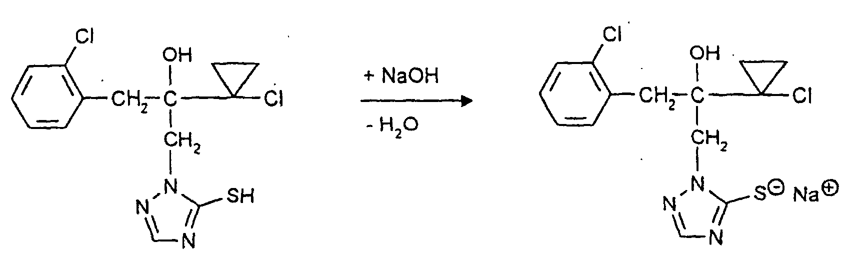

Verwendet man 2-(1-Chlor-cyclopropyl)-1-(2-chlor-phenyl)-3-(5-mercapto-1,2,4-triazol-1-yl)-propan-2-ol als Ausgangsstoff und Triethylamin als Reaktionskomponente, so kann der Verlauf des erfindungsgemäßen Verfahrens (Variante b) durch das folgende Formelschema veranschaulicht werden.

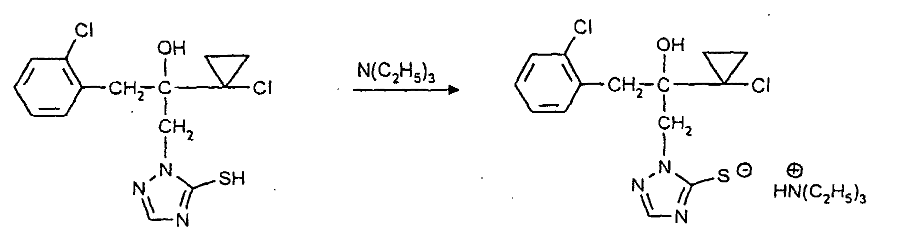

Verwendet man 2-(1-Chlor-cyclopropyl)-1-(2-chlor-phenyl)-3-(5-mercapto-1,2,4-triazol-1-yl)-propan-2-ol als Ausgangsstoff und Kupfer(II)-acetat als Reaktionskomponente, so kann der Verlauf des erfindungsgemäßen Verfahrens (Variante c) durch das folgende Formelschema veranschaulicht werden.

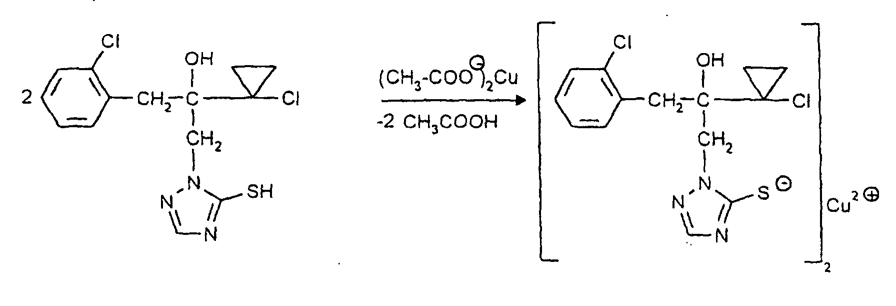

Die bei der Durchführung des erfindungsgemäßen Verfahrens als Ausgangsstoffe benötigten Mercapto-triazole sind durch die Formel (II) allgemein definiert. In dieser Formel hat R¹ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Mercapto-triazole der Formel (II) sind teilweise bekannt (vgl. WO 87-06430). Sie lassen sich herstellen, indem man Triazole der Formel 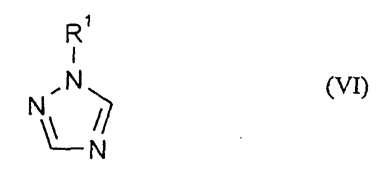 in welcher
- R¹: die oben angegebenen Bedeutungen hat,
entweder
α) nacheinander mit starken Basen und Schwefel in Gegenwart eines Verdünnungsmittels umsetzt und dann mit Wasser, gegebenenfalls in Gegenwart einer Säure hydrolysiert,
   oder
β) mit Schwefel in Gegenwart eines hoch siedenden Verdünnungsmittels umsetzt und dann gegebenenfalls mit Wasser sowie gegebenenfalls mit Säure behandelt.

Die bei der Durchführung des Verfahrens zur Herstellung von Mercapto-triazolen der Formel (II) als Ausgangsstoffe benötigten Triazole sind durch die Formel (VI) allgemein definiert. In dieser Formel hat R¹ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Triazole der Formel (VI) sind bekannt oder lassen sich nach bekannten Methoden herstellen (vgl. EP-A 0 015 756, EP-A 0 040 345, EP-A 0 052 424, EP-A 0 061 835, EP-A 0 297 345, EP-A 0 094 564, EP-A 0 196 038, EP-A 0 267 778, EP-A 0 378 953, EP-A 0 068 813, EP-A 0 044 605, EP-A 0 069 442, EP-A 0 055 833, EP-A 0 301 393, DE-A 2 324 010, DE-A 2 737 489, DE-A 2 551 560, EP-A 0 065 485, DE-A 2 735 872, EP-A 0 234 242, DE-A 2 201 063, EP-A 0 145 294 und DE-A 3 721 786).

Als Basen kommen bei der Durchführung des obigen Verfahrens (α) zur Herstellung von Mercaptotriazolen der Formel (II) alle für derartige Reaktionen üblichen, starken Alkalimetall-Basen in Betracht. Vorzugsweise verwendbar sind n-Butyl-lithium, Lithium-diisopropyl-amid, Natriumhydrid, Natriumamid und auch Kalium-tert.-butylat im Gemisch mit Tetramethylethylen-diamin (= TMEDA).

Bei der Durchführung des obigen Verfahrens (α) zur Herstellung von Mercaptotriazolen der Formel (II) kommen alle für derartige Umsetzungen üblichen inerten organischen Solventien als Verdünnungsmittel in Betracht. Vorzugsweise verwendbar sind Ether, wie Tetrahydrofuran, Dioxan, Diethylether und 1,2-Dimethoxyethan, ferner flüssiger Ammoniak oder auch stark polare Solventien, wie Dimethylsulfoxid.

Schwefel wird sowohl bei der Durchführung des obigen Verfahrens (α) als auch des Verfahrens (β) vorzugsweise in Form von Pulver eingesetzt.

Zur Hydrolyse verwendet man bei der Durchführung des obigen Verfahrens (α) Wasser, gegebenenfalls in Gegenwart einer Säure. In Frage kommen hierbei alle für derartige Umsetzungen üblichen anorganischen oder organischen Säuren. Vorzugsweise verwendbar sind Essigsäure, verdünnte Schwefelsäure und verdünnte Salzsäure. Es ist jedoch auch möglich, die Hydrolyse mit wäßriger Ammoniumchlorid-Lösung durchzuführen.

Die Reaktionstemperaturen können bei der Durchführung des obigen Verfahrens (α) innerhalb eines bestimmten Bereiches variiert werden. Im allgemeinen arbeitet man bei Temperaturen zwischen -70°C und +20°C, vorzugsweise zwischen -70°C und 0°C.

Bei der Durchführung der obigen Verfahren (α) und (β) arbeitet man im allgemeinen unter Normaldruck. Es ist aber auch möglich, unter erhöhtem oder vermindertem Druck zu arbeiten. So kommt vor allem bei der Durchführung des Verfahrens (β) ein Arbeiten unter erhöhtem Druck in Frage.

Bei der Durchführung des obigen Verfahrens (α) setzt man auf 1 Mol an Triazol der Formel (VI) im allgemeinen 2 bis 3 Äquivalente, vorzugsweise 2,0 bis 2,5 Äquivalente, an starker Base und anschließend eine äquivalente Menge oder auch einen Überschuß an Schwefel ein. Die Umsetzung kann unter Schutzgasatmosphäre, z.B. unter Stickstoff oder Argon, vorgenommen werden. Die Aufarbeitung erfolgt nach üblichen Methoden. Im allgemeinen geht man so vor, daß man das Reaktionsgemisch mit einem in Wasser wenig löslichen organischen Solvens extrahiert, die vereinigten organischen Phasen trocknet und einengt und den verbleibenden Rückstand gegebenenfalls durch Umkristallisation und/oder Chromatographie reinigt.

Bei der Durchführung des obigen Verfahrens (β) kommen als Verdünnungsmittel alle für derartige Umsetzungen üblichen, hoch siedenden organischen Solventien in Betracht. Vorzugsweise verwendbar sind Amide, wie Dimethylformamid und Dimethylacetamid, außerdem heterocyclische Verbindungen, wie N-Methyl-pyrrolidon, und auch Ether, wie Diphenylether.

Bei der Durchführung des obigen Verfahrens (β) kann nach der Umsetzung gegebenenfalls eine Behandlung mit Wasser sowie gegebenenfalls mit Säure vorgenommen werden. Diese wird so durchgeführt, wie die Hydrolyse bei der Durchführung des Verfahrens (α).

Die Reaktionstemperaturen können auch bei der Durchführung des obigen Verfahrens (β) innerhalb eines größeren Bereiches variiert werden. Im allgemeinen arbeitet man bei Temperaturen zwischen 150°C und 300°C, vorzugsweise zwischen 180°C und 250°C.

Bei der Durchführung des obigen Verfahrens (β) setzt man auf 1 Mol Triazol der Formel (VI) im allgemeinen 1 bis 5 Mol, vorzugsweise 1,5 bis 3 Mol an Schwefel ein. Die Aufarbeitung erfolgt nach üblichen Methoden. Im allgemeinen geht man so vor, daß man das Reaktionsgemisch mit einem in Wasser nur wenig löslichen organischen Solvens extrahiert, die vereinigten organischen Phasen trocknet und einengt und den verbleibenden Rückstand gegebenenfalls nach üblichen Methoden, wie Umkristallisation oder Chromatographie, von eventuell vorhandenen Verunreinigungen befreit.

Die bei der Durchführung des erfindungsgemäßen Verfahrens nach Variante (a) als Reaktionskomponenten benötigten Hydroxide sind durch die Formel (III) allgemein definiert. In dieser Formel steht
- M¹: steht bevorzugt für ein Natrium- oder Kalium-Kation, ein Äquivalent eines Magnesium- oder Calcium-Kations oder für ein Ammonium-Kation der Formel 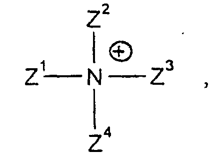 in welcher
Z¹, Z², Z³ und Z⁴ bevorzugt diejenigen Bedeutungen haben, die bereits im Zusammenhang mit der Beschreibung der erfindungsgemäßen Stoffe der Formel (I) als besonders bevorzugt für diese Substituenten genannt wurden.

Die Hydroxide der Formel (III) sind bekannt oder lassen sich nach bekannten Methoden herstellen.

Als Verdünnungsmittel kommen bei der Durchführung des erfindungsgemäßen Verfahrens nach Variante (a) alle üblichen inerten, organischen Solventien in Betracht. Vorzugsweise verwendbar sind halogenierte Kohlenwasserstoffe, wie Dichlormethan, ferner Alkohole, wie Methanol, Ethanol, n-Propanol oder n-Butanol, und weiterhin stark polare Lösungsmittel, wie Dimethylformamid oder Dimethylsulfoxid.

Die Reaktionstemperaturen können bei der Durchführung des erfindungsgemäßen Verfahrens nach Variante (a) innerhalb eines größeren Bereiches variiert werden. Im allgemeinen arbeitet man bei Temperaturen zwischen -30°C und +100°C, vorzugsweise bei Temperaturen zwischen 0°C und +60°C.

Bei der Durchführung des erfindungsgemäßen Verfahrens sowohl nach Variante (a) als auch nach den Varianten (b) oder (c) arbeitet man im allgemeinen unter Atmosphärendruck. Es ist jedoch auch möglich, unter erhöhtem oder vermindertem Druck zu arbeiten.

Bei der Durchführung des erfindungsgemäßen Verfahrens nach Variante (a) setzt man auf 1 Mol an Mercapto-triazol der Formel (II) im allgemeinen eine äquivalente Menge oder auch einen Überschuß an Hydroxid der Formel (III) ein. Die Aufarbeitung erfolgt nach üblichen Methoden. Im allgemeinen geht man so vor, daß man das Reaktionsgemisch unter vermindertem Druck einengt und den Rückstand gegebenenfalls wäscht und trocknet. Das erhaltene Produkt kann gegebenenfalls nach üblichen Methoden von eventuell noch vorhandenen Verunreinigungen befreit werden.

Die bei der Durchführung des erfindungsgemäßen Verfahrens nach Variante (b) als Reaktionskomponenten benötigten Amine sind durch die Formel (IV) allgemein definiert. In dieser Formel haben Z², Z³ und Z⁴ diejenigen Bedeutungen, die bereits im Zusammenhang mit der Beschreibung der erfindungsgemäßen Stoffe der Formel (I) für diese Substituenten genannt wurden.
- Z²: steht bevorzugt für Wasserstoff oder geradkettiges oder verzweigtes Alkyl mit 1 bis 6 Kohlenstoffatomen,
- Z³: steht bevorzugt für Wasserstoff, geradkettiges oder verzweigtes Alkyl mit 1 bis 6 Kohlenstoffatomen, Benzyl oder Phenyl, und
- Z⁴: steht besonders bevorzugt für geradkettiges oder verzweigtes Alkyl mit 1 bis 18 Kohlenstoffatomen, Benzyl oder Phenyl.

Die Amine der Formel (IV) sind bekannt oder lassen sich nach bekannten Methoden herstellen.

Als Verdünnungsmittel kommen bei der Durchführung des erfindungsgemäßen Verfahrens nach Variante (b) alle üblichen, inerten organischen Solventien in Betracht. Vorzugsweise verwendbar sind aromatische Kohlenwasserstoffe, wie Toluol, Xylol oder Decalin, ferner halogenierte Kohlenwasserstoffe, wie Dichlormethan, Chloroform, Tetrachlormethan, Dichlorethan oder Trichlorethan, außerdem Ether, wie Diethylether, Diisopropylether, Methyl-tert.-butylether, Methyl-tert.amylether, Dioxan oder Tetrahydrofuran, und weiterhin Nitrile, wie Acetonitril, Propionitril, n- oder iso-Butyronitril.

Die Reaktionstemperaturen können auch bei der Durchführung des erfindungsgemäßen Verfahrens nach Variante (b) innerhalb eines bestimmten Bereiches variiert werden. Im allgemeinen arbeitet man bei Temperaturen zwischen 0°C und 100°C, vorzugsweise zwischen 10°C und 60°C.

Bei der Durchführung des erfindungsgemäßen Verfahrens nach Variante (b) setzt man auf 1 Mol an Mercapto-triazol der Formel (II) im allgemeinen eine äquivalente Menge oder auch einen Überschuß an Amin der Formel (IV) ein. Die Aufarbeitung erfolgt nach üblichen Methoden. Im allgemeinen geht man so vor, daß man das Reaktionsgemisch unter vermindertem Druck einengt und den Rückstand gegebenenfalls wäscht und trocknet. Das erhaltene Produkt kann nach üblichen Methoden von eventuell noch vorhandenen Verunreinigungen befreit werden.

Die bei der Durchführung des erfindungsgemäßen Verfahrens nach Variante (c) als Reaktionskomponenten benötigten Acetate sind durch die Formel (V) definiert.

Die Acetate der Formel (V) sind bekannt.

Als Verdünnungsmittel kommen bei der Durchführung des erfindungsgemäßen Verfahrens nach Variante (c) wiederum alle für derartige Umsetzungen üblichen, inerten organischen Solventien in Frage. Vorzugsweise verwendbar sind diejenigen Lösungsmittel, die bereits im Zusammenhang mit der Beschreibung der Verfahrensvariante (a) als bevorzugt genannt wurden.

Die Reaktionstemperaturen können auch bei der Durchführung des erfindungsgemäßen Verfahrens nach Variante (c) innerhalb eines bestimmten Bereiches variiert werden. Im allgemeinen arbeitet man bei Temperaturen zwischen 0°C und 100°C, vorzugsweise zwischen 10°C und 60°C.

Bei der Durchführung des erfindungsgemäßen Verfahren nach Variante (c) setzt man auf 1 Mol an Mercapto-triazol der Formel (II) im allgemeinen eine äquivalente Menge oder auch einen Überschuß an Acetat der Formel (V) ein. Die Aufarbeitung erfolgt nach üblichen Methoden. Im allgemeinen geht man so vor, daß man das Reaktionsgemisch unter vermindertem Druck einengt und den Rückstand gegebenenfalls wäscht und trocknet. Das erhaltene Produkt kann nach üblichen Methoden von eventuell noch vorhandenen Verunreinigungen befreit werden.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Pseudocercosporella, Erysiphe- und Fusarium-Arten. Außerdem lassen sich die erfindungsgemäßen Stoffe sehr gut gegen Venturia und Sphaerotheca einsetzen. Sie besitzen darüber hinaus auch eine sehr gute in-vitro Wirkung.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole,. Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder. Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung im Pflanzenschutz als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen beispielsweise die folgenden Stoffe infrage.

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid;(E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl }-3-methoxyacrylat; Methyl-(E)-methoximino [alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Technazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl,. Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden, Diese Mischungen können ein breiteres Wirkungsspektrum besitzen als die erfindungsgemäßen Verbindungen.

Die Herstellung und die Verwendung der erfindungsgemäßen Stoffe gehen aus den folgenden Beispielen hervor.

### Herstellungsbeispiele

### Beispiel 1

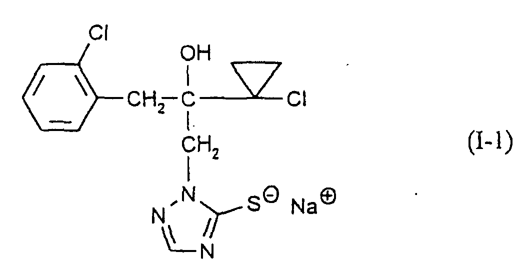

Eine Lösung von 50 g (0,146 Mol) 2-(1-Chlor-cyclopropyl)-1-(2-chlorphenyl)-3-(5-mercapto-1,2,4-triazol-1-yl)-propan-2-ol in 730 ml absolutem Ethanol wird unter Rühren bei Raumtemperatur tropfenweise mit einer Lösung von 5,84 g (0,146 Mol) Natriumhydroxid in 365 ml absolutem Ethanol versetzt. Nach beendeter Zugabe wird das Reaktionsgemisch unter vermindertem Druck eingeengt. Der verbleibende Rückstand wird mit n-Hexan gewaschen und getrocknet. Man erhält auf diese Weise 52,8 g (98 % der Theorie) an Natriumsalz des 2-(1-Chlorcyclopropyl)-1-(2-chlorphenyl)-3-(5-mercapto-1,2,4-triazol-1-yl)-propan-2-ols in Form einer Festsubstanz vom Schmelzpunkt 268-270°C.

### Beispiel 2

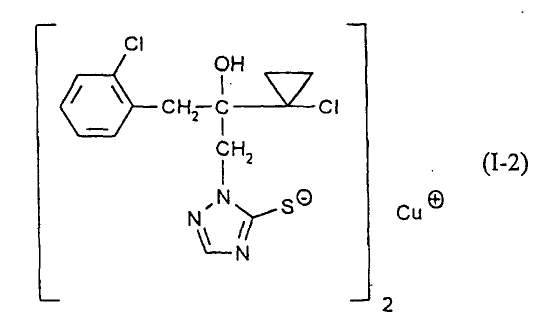

Eine Lösung von 1,72 g (5 mMol) 2-(1-Chlor-cyclopropyl)-1-(2-chlorphenyl)-3-(5-mercapto-1,2,4-triazol-1-yl)-propan-2-ol in 20 ml absolutem Methanol wird bei Raumtemperatur unter Rühren tropfenweise mit einer Lösung von 0,5 g (2,5 mMol) Kupfer(II)-acetat in 5 ml Wasser versetzt. Nach beendeter Zugabe wird das Reaktionsgemisch unter Rühren 2 Stunden auf 50°C erhitzt. Anschließend wird das Reaktionsgemisch unter vermindertem Druck eingeengt. Der verbleibende Rückstand wird im Hochvakuum getrocknet. Man erhält auf diese Weise 1,9 g (100 % der Theorie) an Kupfersalz des 2-(1-Chlor-cyclopropyl)-1-(2-chlorphenyl)-3-(5-mercapto-1,2,4-triazol-1-yl)-propan-2-ols in Form einer Festsubstanz vom Schmelzpunkt 107-109°C.

### Beispiel 3

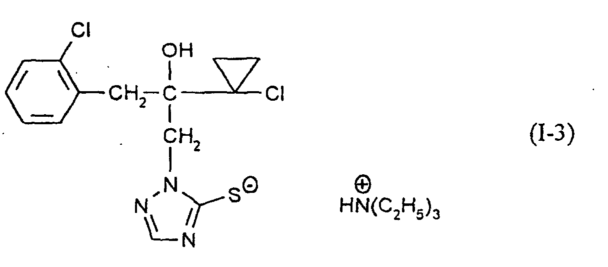

Eine Lösung von 1,72 g (5 mMol) 2-(1-Chlorcyclopropyl)-1-(2-chlorphenyl)-3-(5-mercapto-1,2,4-triazol-1-yl)-propan-2-ol in 20 ml Dichlormethan wird bei Raumtemperatur unter Rühren tropfenweise mit 0,5 g (5 mMol) Triethylamin versetzt. Nach beendeter Zugabe wird das Reaktionsgemisch zunächst eine Stunde bei 20°C nachgerührt und dann unter vermindertem Druck eingeengt. Der verbleibende Rückstand wird im Hochvakuum getrocknet. Man erhält auf diese Weise 2,2 g (99 % der Theorie) an Triethylammonium-salz des 2-(1-Chlor-cyclopropyl)-1-(2-chlorphenyl)-3-(5-mercapto-1,2,5-triazol-1-yl)-propan-2-ols in Form eines Öles.
¹H-NMR-Spektrum (200 MHz; CDCl₃; TMS):
- δ =: 0,6-0,9 (m, 4H); 1,3 (t, 9 H); 3,2 (q, 6H); 3,35 (AB, 2H); 4,65 (AB, 2H); 5,9 (s, OH); 7,1-7,65 (m, 4H); 7,6 (s, 1H); 11,7 (NH) ppm.

Nach der im Beispiel 3 angegebenen Methode werden auch die in den folgenden Beispielen aufgeführten Verbindungen hergestellt.

### Beispiel 4

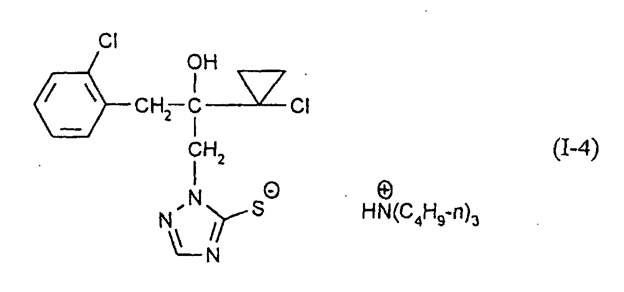

Öl
¹H-NMR-Spektrum (200 MHz; CDCl₃, TMS):
- δ =: 0,6-1,0 (m, 4H); 0,95 (t, 9H); 1,3-1,7 (m, 12H), 3,0 (m, 6H); 3,15 (d, 1H); 3,55 (d, 1H); 4,55 (d, 1H); 4,75 (d, 1H); 6,05 (OH); 7,1-7,65 (m, 4H); 7,55 (s, 1H); 11,6 (NH) ppm.

### Beispiel 5

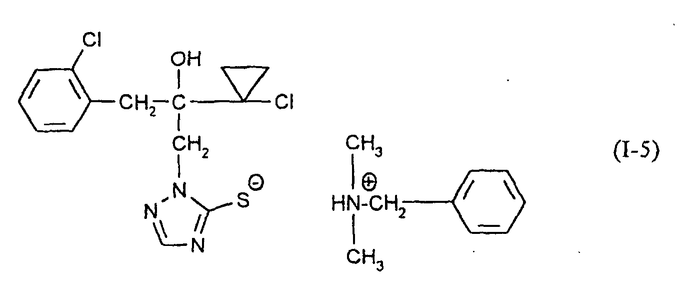

Schmelzpunkt: 86°C.

### Beispiel 6

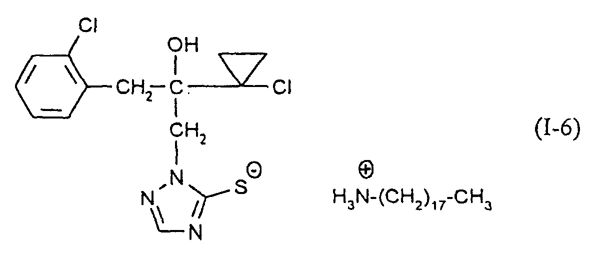

¹H-NMR-Spektrum (400 MHz; CDCl₃; TMS):
- δ =: 0,7-0,9 (m, 4H); 0,9 (t, 9H); 1,25 (m, 30H); 1,5 (m, 3H); 2,8 (t, 2H); 3,2 (d, 1H); 3,5 (d, 1H); 4,5 (d, 1H); 4,75 (d, 1H); 6,2 (OH); 7,5-7,55 (m, 4H); 7,65 (s, 1H) ppm.

### Beispiel 7

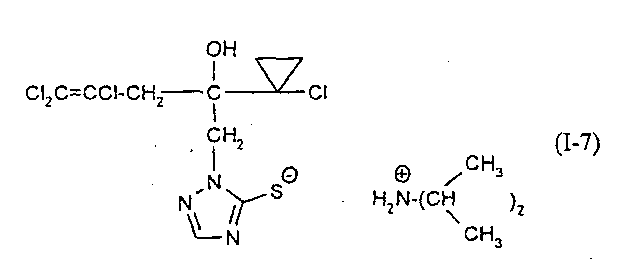

Schmelzpunkt: 132-135°C.

### Beispiel 8

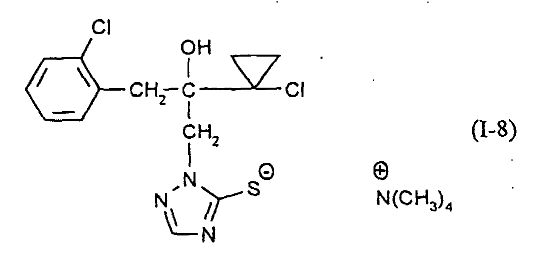

1,72 g (5 mMol) 2-(1-Chlor-cyclopropyl)-1-(2-chlorphenyl)-3-(5-mercapto-1,2,4-triazol-1-yl)-propan-2-ol und 0,91 g (5 mMol) Tetramethylammonium-hydroxid in Form des Pentahydrates werden in 30 ml Dichlormethan unter Rühren bei Raumtemperatur gelöst. Anschließend wird das Reaktionsgemisch unter vermindertem Druck eingeengt. Der verbleibende Rückstand wird im Hochvakuum getrocknet. Man erhält auf diese Weise 2,1 g (100 % der Theorie) an Tetramethylammoniumsalz des 2-(1-Chlorcyclopropyl)-1-(2-chlorphenyl)-3-(5-mercapto-1,2,4-triazol-1-yl)-propan-2-ols in Form einer Festsubstanz vom Schmelzpunkt 155°C.

### Herstellung von Ausgangssubstanzen:

### Beispiel 9

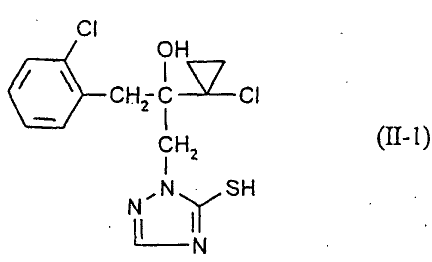

### Variante α:

Ein Gemisch aus 3,12 g (10 mMol) 2-(1-Chlor-cyclopropyl)-1-(2-chlorphenyl)-3-(1,2,4-triazol-1-yl)-propan-2-ol und 45 ml absolutem Tetrahydrofuran wird bei -20°C mit 8,4 ml (21 mMol) n-Butyl-lithium in Hexan versetzt und 30 Minuten bei 0°C gerührt. Danach wird das Reaktionsgemisch auf -70°C abgekühlt, mit 0,32 g (10 mMol) Schwefel-Pulver versetzt und 30 Minuten bei -70°C gerührt. Es wird auf -10°C erwärmt, mit Eiswasser versetzt und durch Zugabe von verdünnter Schwefelsäure auf einen pH-Wert von 5 eingestellt. Man extrahiert mehrfach mit Essigsäureethylester, trocknet die vereinigten organischen Phasen über Natriumsulfat und engt unter vermindertem Druck ein. Man erhält auf diese Weise 3,2 g (93 % der Theorie) an 2-(1-Chlor-cyclopropyl)-1-(2-chlorphenyl)-3-(5-mercapto-1,2,4-triazol-1-yl)-propan-2-ol in Form einer Festsubstanz, die nach Umkristallisation bei 138-139°C schmilzt.

### Variante β:

Ein Gemisch aus 3,12 g (10 mmol) 2-(1-Chlor-cyclopropyl)-1-(2-chlor-phenyl)-3-(1,2,4-triazol-1-yl)-propan-2-ol, 0,96 g (30 mmol) Schwefel-Pulver und 20 ml absolutem N-Methyl-pyrrolidon wird unter Rühren 44 Stunden auf 200°C erhitzt. Anschließend wird das Reaktionsgemisch unter vermindertem Druck (0,2 mbar) eingeengt. Das dabei anfallende Rohprodukt (3,1 g) wird aus Toluol umkristallisiert. Man erhält auf diese Weise 0,7 g (20 % der Theorie) an 2-(1-Chlorcyclopropyl)-1-(2-chlorphenyl)-3-(5-mercapto-1,2,4-triazol-1-yl)-propan-2-ol in Form einer Festsubstanz vom Schmelzpunkt 138-139°C.

### Beispiel 10

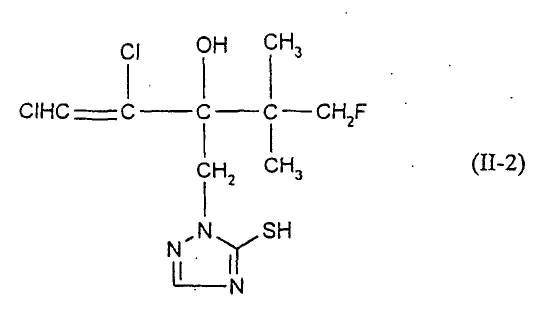

Ein Gemisch aus 1,41 g (5 mMol) 1,2-Dichlor-4,4-dimethyl-5-fluor-3-hydroxy-3-[(1,2,4-triazol-1-yl)-methyl]-1-penten und 25 ml absolutem Tetrahydrofuran wird bei -70°C mit 4 ml (10 mMol) n-Butyl-lithium in Hexan versetzt und eine Stunde bei -70°C gerührt. Danach wird das Reaktionsgemisch mit 0,19 g (6 mMol) Schwefel-Pulver versetzt und 4 Stunden bei -70°C gerührt. Anschließend wird hydrolysiert, indem man 1 ml Methanol und 1 ml Essigsäure bei -70°C hinzufügt. Das Reaktionsgemisch wird zunächst mit Essigsäureethylester verdünnt und dann mehrfach mit gesättigter, wäßriger Ammoniumchlorid-Lösung ausgeschüttelt. Die organische Phase wird über Natriumsulfat getrocknet und unter vermindertem Druck eingeengt. Das anfallende Rohprodukt (1,7 g) wird durch Chromatographie an Kieselgel mit einem Gemisch aus Petrolether und Essigsäureethylester = 1:1 als Laufmittel gereinigt. Man erhält auf diese Weise 0,5 g (32 % der Theorie) an 1,2-Dichlor-4,4-dimethyl-5-fluor-3-hydroxy-3-[(5-mercapto-1,2,4-triazol-1-yl)-methyl]-1-penten in Form einer Festsubstanz vom Schmelzpunkt 162-164°C.

Nach den in den Beispielen 9 und 10 angegebenen Methoden werden auch die in der folgenden Tabelle 2 aufgeführten Verbindungen hergestellt.

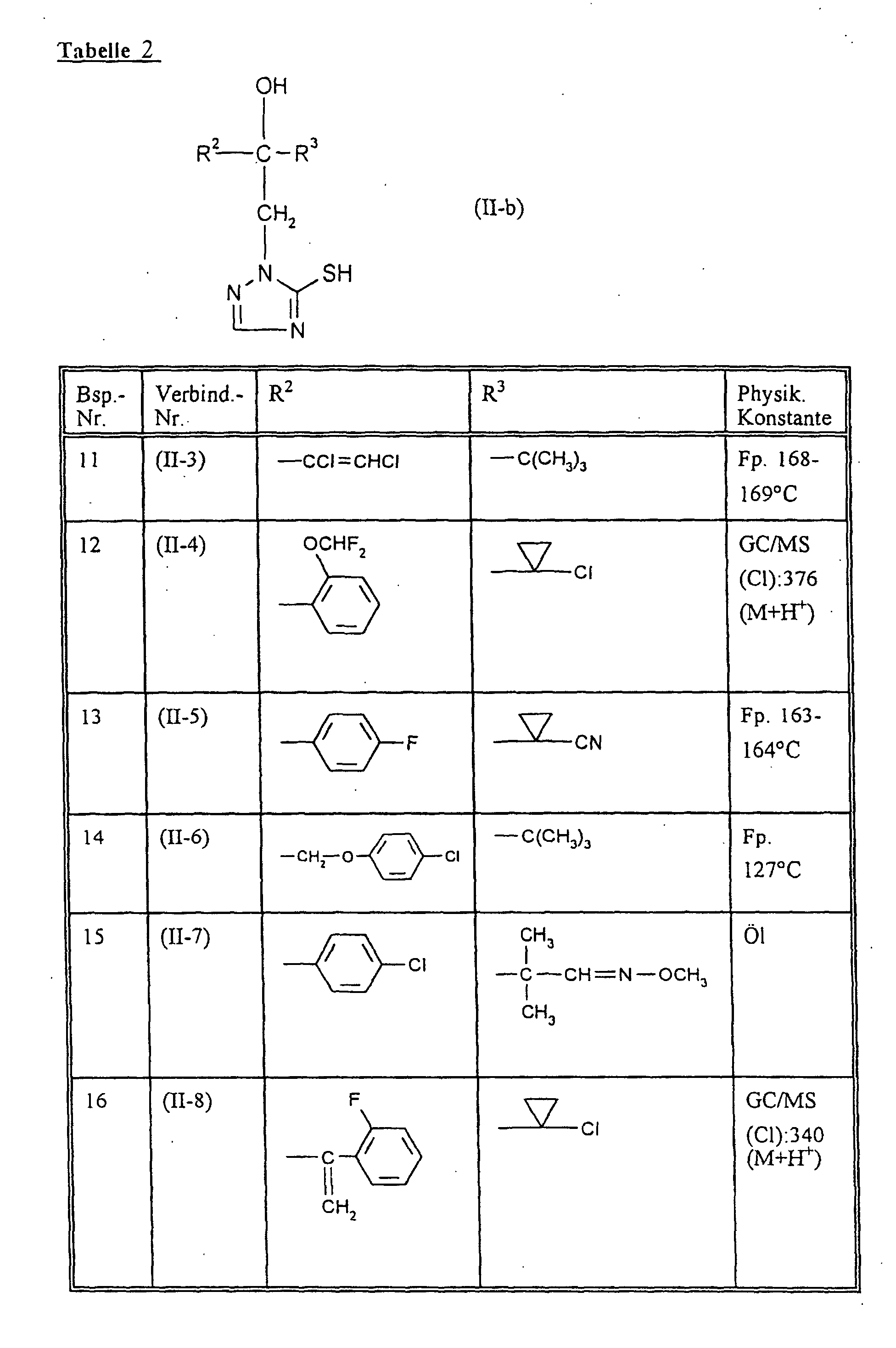

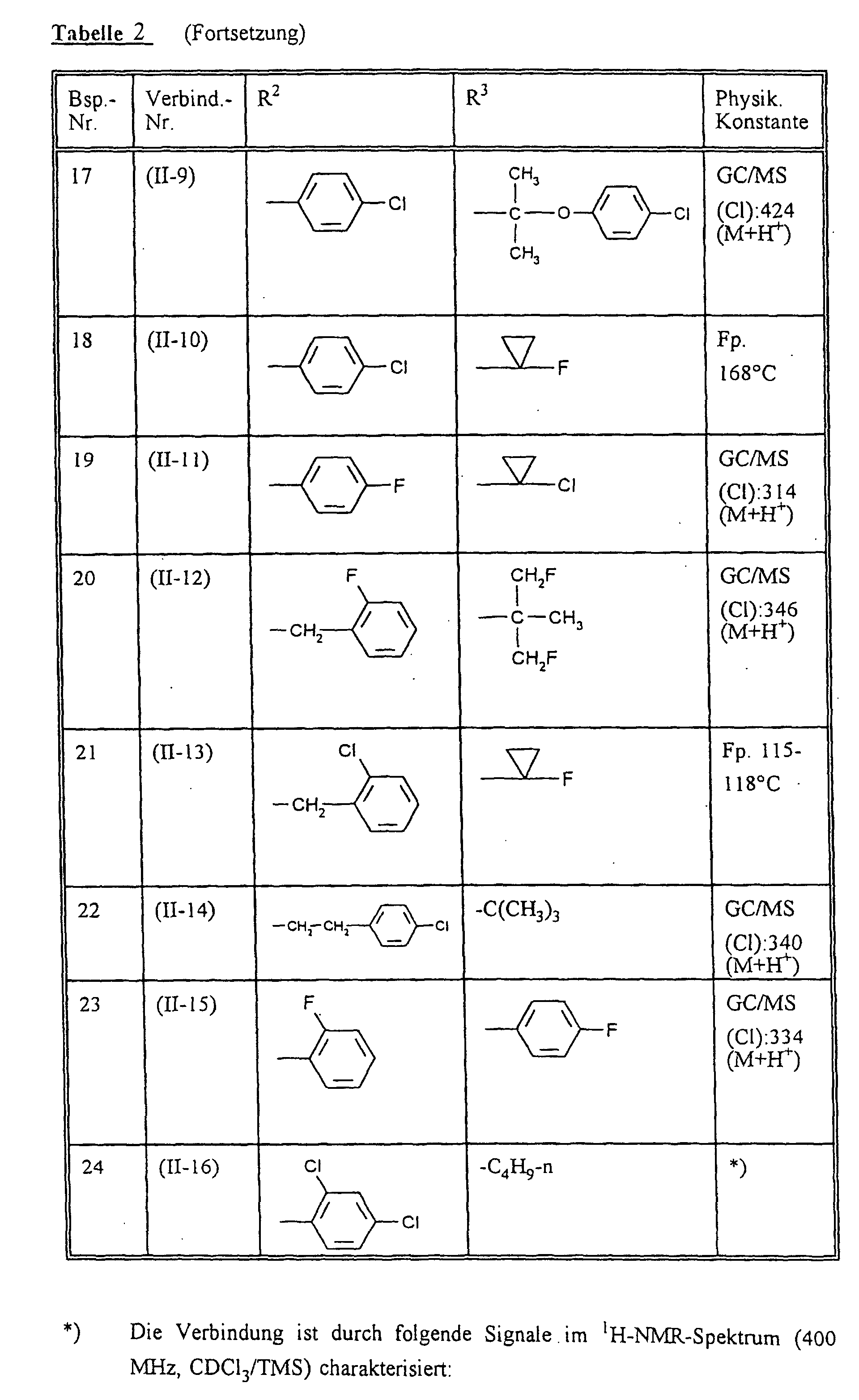

- δ =: 0,8 (t, 3H); 0,85 (m, 2H); 1,25 (m, 2H); 1,8 (m, 1H); 2,55 (m, 1H); 4,6 (OH); 4,9 (AB, 2H); 7,2 (dd, 1H); 7,35 (d, 1H); 7,7 (s, 1H); 7,75 (d, 1H); 12,3 (5H) ppm

### Beispiel A

### Erysiphe-Test (Gerste) / kurativ

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

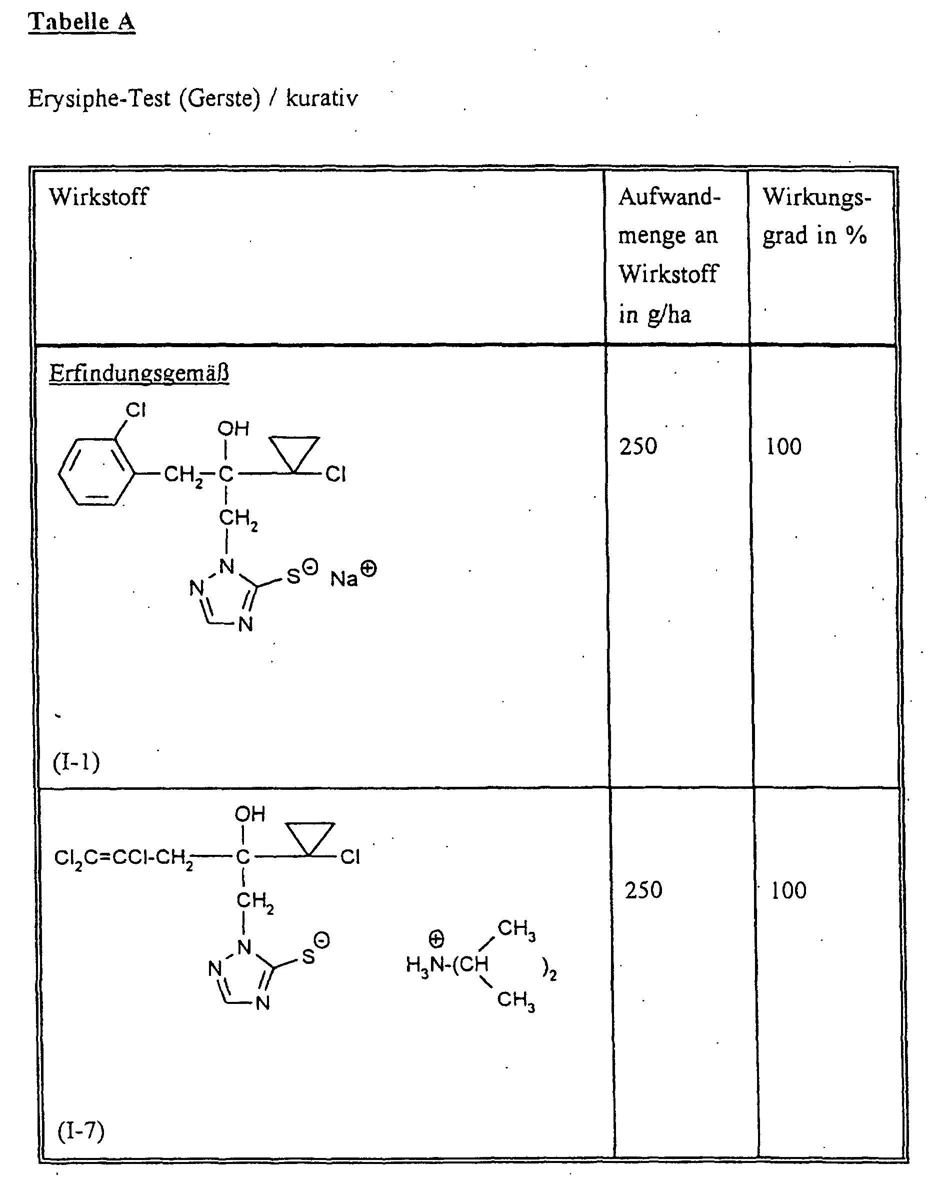

### Beispiel B

### Erysiphe-Test (Gerste) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

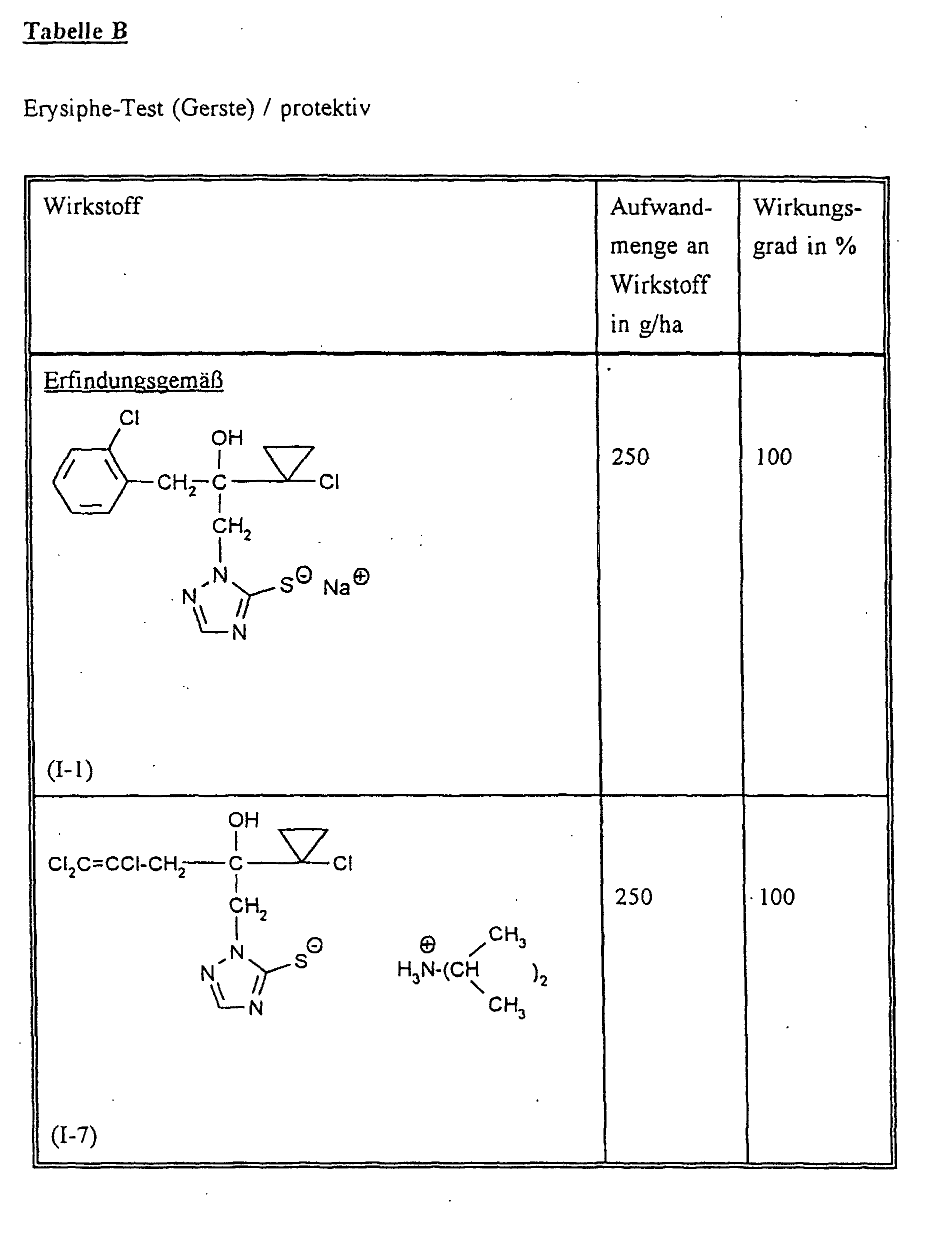

### Beispiel C

### Erysiphe-Test (Weizen) / kurativ

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. tritici bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

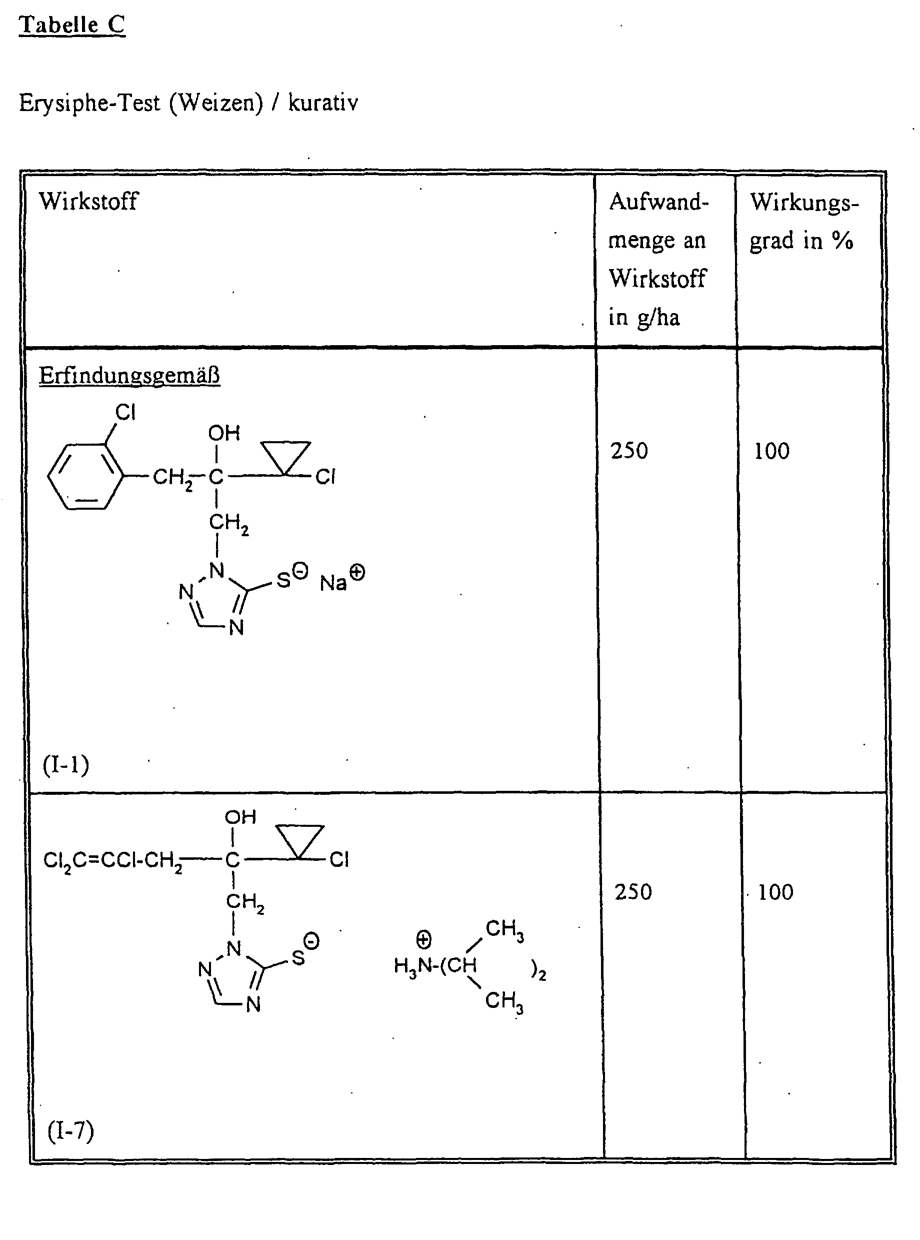

### Beispiel D

### Erysiphe-Test (Weizen) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

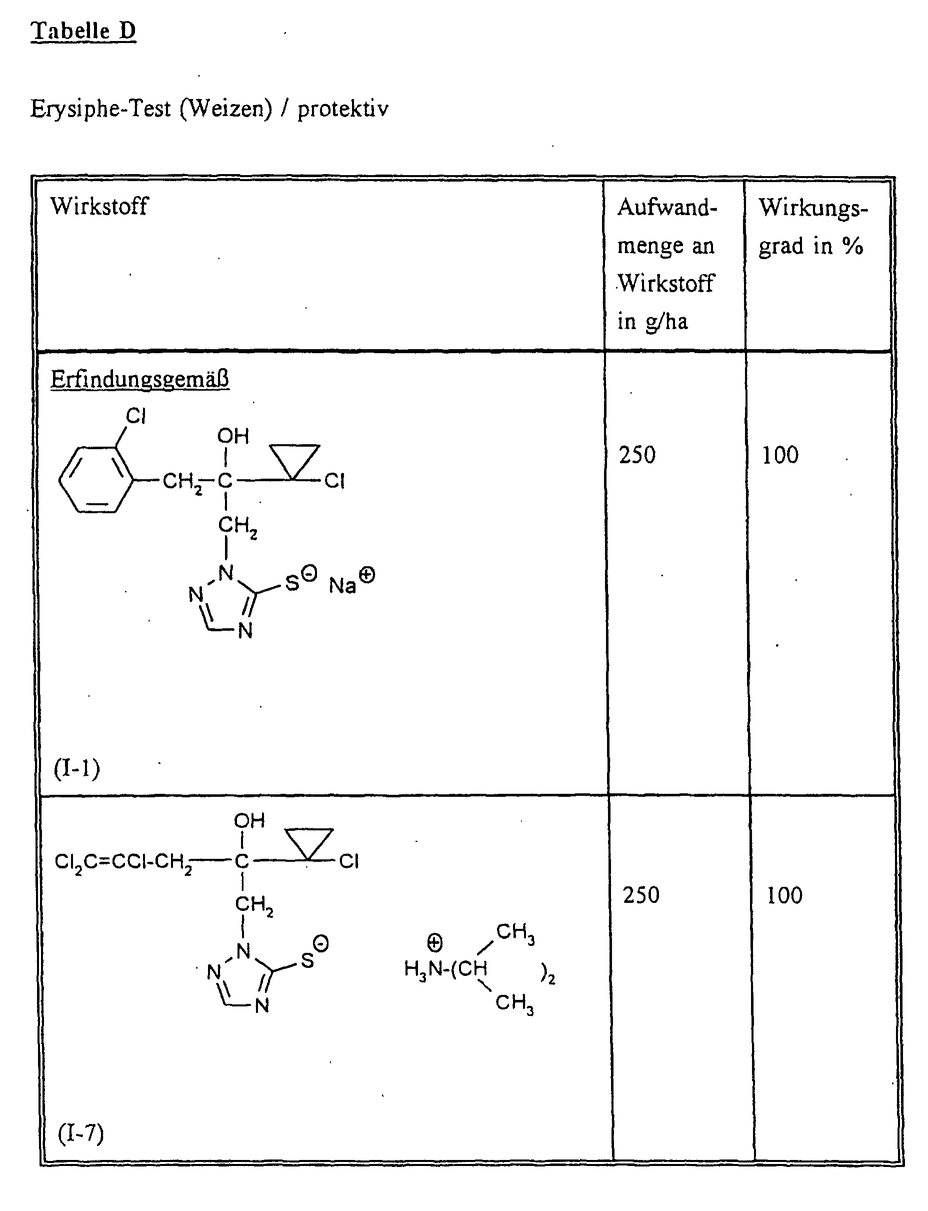

### Beispiel E

### Sphaerotheca-Test (Gurke) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

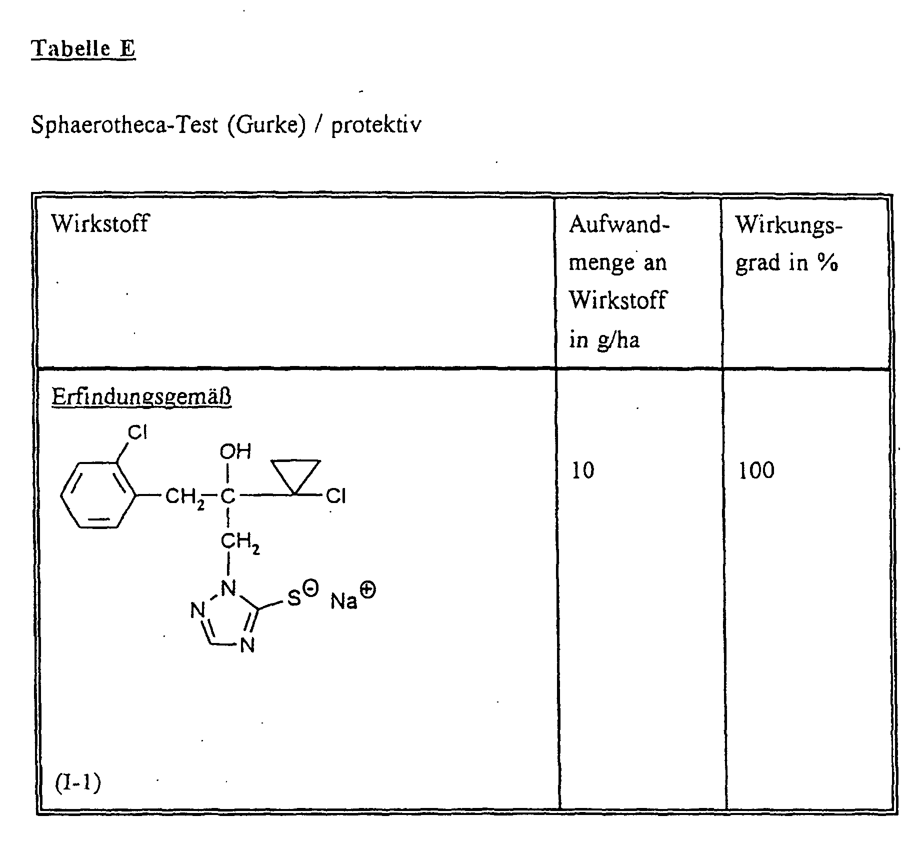

### Beispiel F

### Botrytis-Test (Bohne) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine, mit Botrytis cinerea bewachsene Agarstückchen aufgelegt. Die inokulierten Pflanzen werden in einer abgedunkelten, feuchten Kammer bei 20°C aufgestellt.

3 Tage nach der Inokulation wird die Größe der Befallsflecken auf den Blättern ermittelt und in % ausgedrückt. Dabei bedeutet 0 % ein Wirkungsgrad, der demjenigen der Kontrolle entspricht, während ein Wirkungsgrad von 100% bedeutet, daß kein Befall beobachtet wird.

Wirkstoffe, Wirkstoffkonzentrationen und Versuchsergebnisse gehen aus der folgenden Tabelle hervor.

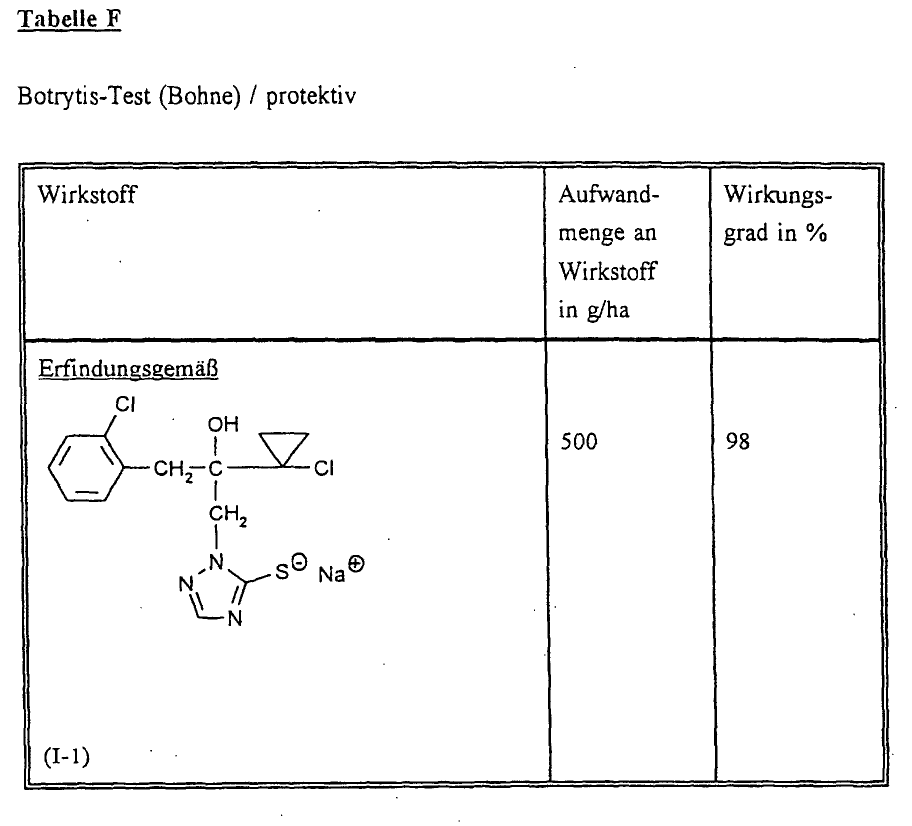

### Beispiel G

### Podosphaera-Test (Apfel) protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Apfelmehltauerregers Podosphaera leucotricha inokuliert. Die Pflanzen werden im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

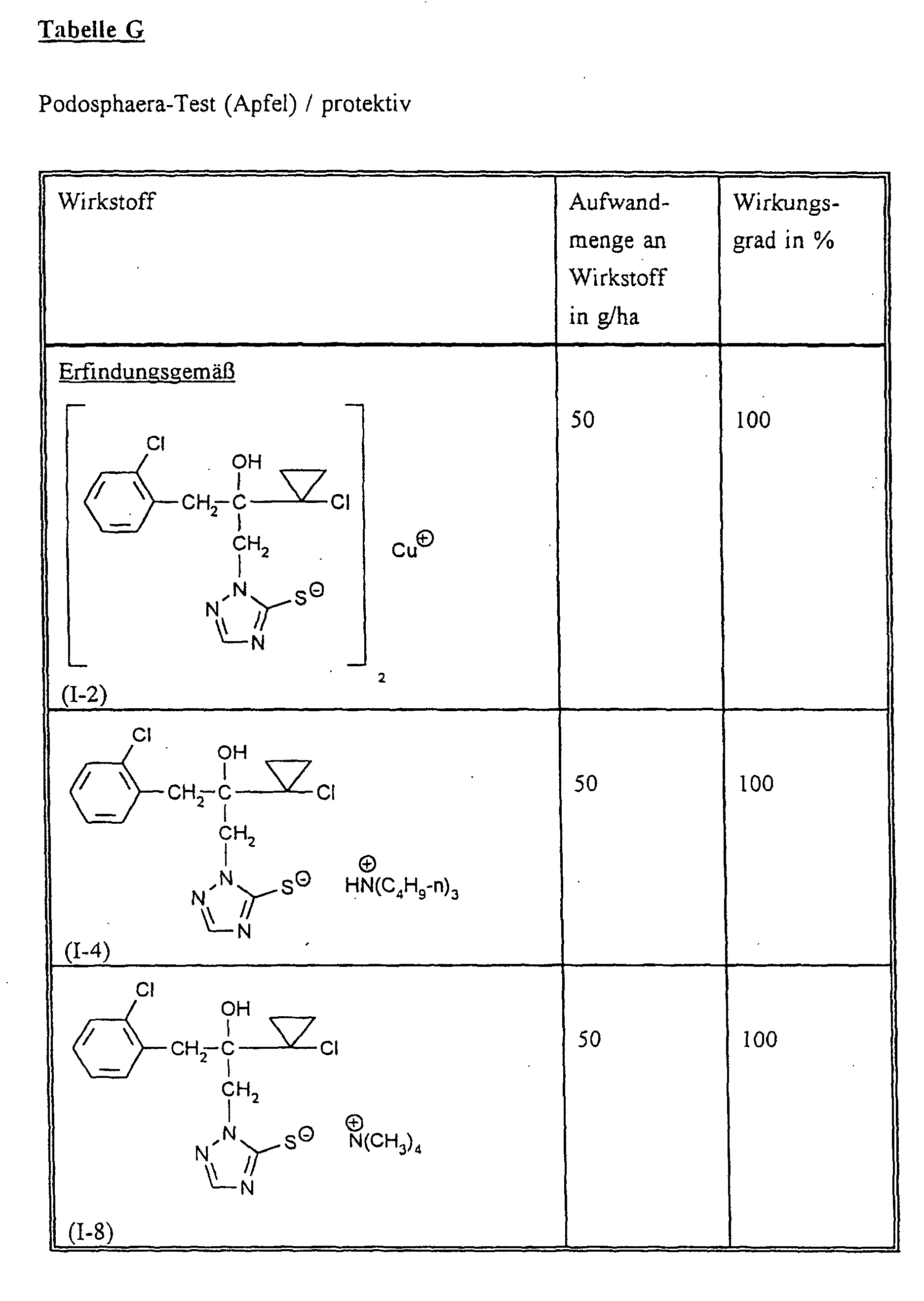

### Beispiel H

### Venturia-Test (Apfel) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentration und Versuchsergebnisse gehen aus der folgenden Tabelle hervor.

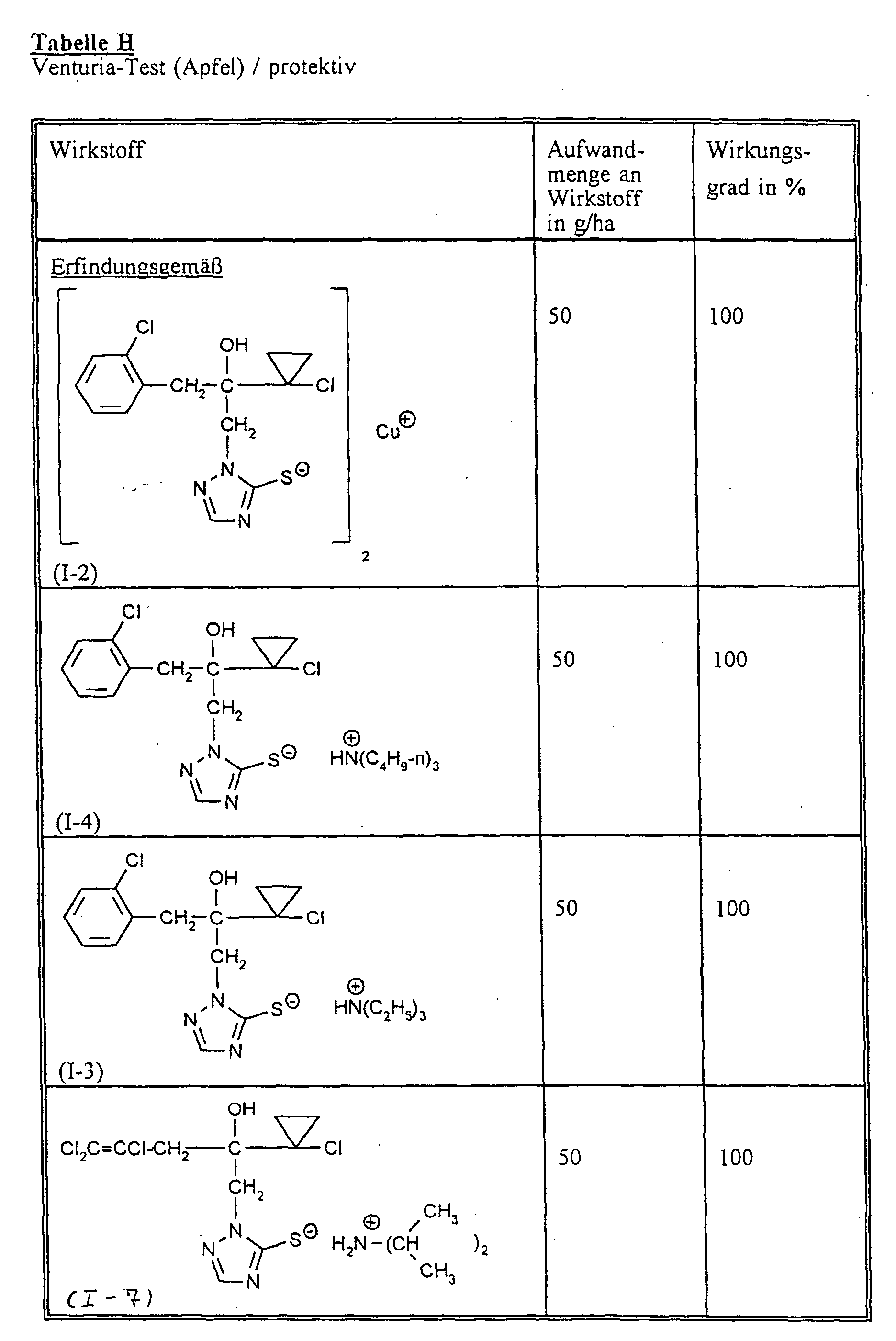

## Patentansprüche

1. Triazolyl-mercaptide der Formel 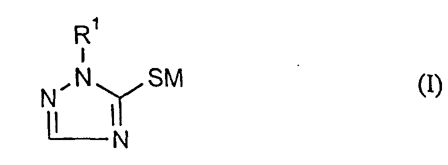 in welcher
M für ein Natrium- oder Kalium-Kation, ein Äquivalent eines Magnesium-, Calcium-, Kupfer-, Zink-, Eisen- oder Nickel-Kations oder für ein Ammonium-Kation der Formel 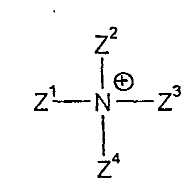 steht, in welcher
Z¹ für Wasserstoff oder geradkettiges oder verzweigtes Alkyl mit 1 bis 8 Kohlenstoffatomen steht,
Z² für Wasserstoff oder geradkettiges oder verzweigtes Alkyl mit 1 bis 8 Kohlenstoffatomen steht,
Z³ für Wasserstoff, geradkettiges oder verzweigtes Alkyl mit 1 bis 8 Kohlenstoffatomen, Benzyl oder Phenyl steht und
Z⁴ für geradkettiges oder verzweigtes Alkyl mit 1 bis 18 Kohlenstoffatomen, Benzyl oder Phenyl steht,
und
R¹ für einen Rest der Formel 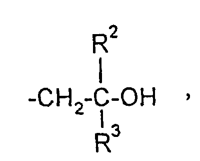 steht, in welcher
R² für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für geradkettiges oder verzweigtes Arkenyl mit 2 bis 6 Kohlenstoffatomen steht, wobei jeder dieser Reste einfach bis dreifach, gleichartig oder verschieden substituiert sein kann durch Halogen, Alkoxy mit 1 bis 4 Kohlenstoffatomen und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aralkenyl mit 6 bis 10 Kohlenstoffatomen im Arylteil und 2 bis 4 Kohlenstoffatomen im Alkenylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4
Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1-bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano, und
R³ für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für geradkettiges oder verzweigtes Alkenyl mit 2 bis 6 Kohlenstoffatomen steht, wobei jeder dieser Reste einfach bis dreifach, gleichartig oder verschieden substituiert sein kann durch Halogen, Alkoxy mit 1 bis 4 Kohlenstoffatomen und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aralkenyl mit 6 bis 10 Kohlenstoffatomen im Arylteil und 2 bis 4 Kohlenstoffatomen im Alkenylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano

2. Verfahren zur Herstellung von Triazolyl-mercaptiden der Formel (I) gemäß Anspruch 1, **dadurch gekennzeichnet, daß** man Mercapto-triazole der Formel 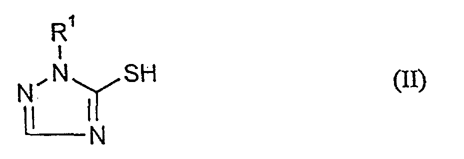 in welcher
R¹ die oben angegebenen Bedeutungen hat,
entweder
a) mit Hydroxiden der Formel
M¹-OH (III)
in welcher
M¹ für , Natrium- oder Kalium-Kation, ein Äquivalent eines Magnesium- oder Calcium-Kations oder für ein Ammonium-Kation der Formel 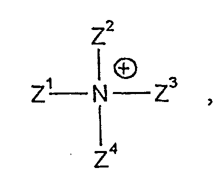
in welcher
Z¹, Z², Z³ und Z⁴ die oben angegebenen Bedeutungen haben, in Gegenwart eines Verdünnungsmittels umsetzt,
oder
b) mit Aminen der Formel 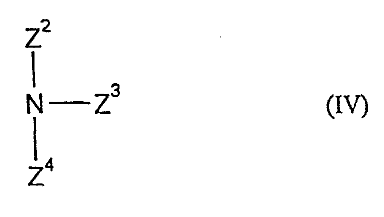 in welcher
Z², Z³ und Z⁴ die oben angegebenen Bedeutungen haben,
in Gegenwart eines Verdünnungsmittels umsetzt,
oder
c) mit Acetaten der Formel
CH₃-COO M² (V)
in welcher
M² für ein Äquivalent eines Kupfer-, Zink-, Eisen- oder Nickel-Kations steht,
in Gegenwart eines Verdünnungsmittels umsetzt.

3. Mikrobizide Mittel, **gekennzeichnet durch** einen Gehalt an mindestens einem Triazolyl-mercaptid der Formel (I) gemäß Anspruch 1 neben Streckmitteln und / oder oberflächenaktiven Stoffen.

4. Verwendung von Triazolyl-mercaptiden der Formel (I) gemäß Anspruch 1 als Mikrobizide im Pflanzenschutz und im Materialschutz.

5. Verfahren zur Bekämpfung von unerwünschten Mikroorganismen im Pflanzenschutz und im Materialschutz, **dadurch gekennzeichnet, daß** man Triazolyl-mercaptide der Formel (I) gemäß Anspruch 1 auf die Mikroorganismen und/oder deren Lebensraum ausbringt.

6. Verfahren zur Herstellung von mikrobiziden Mitteln, **dadurch gekennzeichnet, daß** man Triazolyl-mercaptide der Formel (I) gemäß Anspruch 1 mit Streckmitteln und/oder oberflächenaktiven Stoffen vermischt.

## Claims

1. Triazolyl-mercaptides of the formula 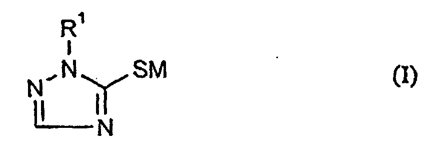 in which
M represents a sodium or potassium cation, an equivalent of a magnesium, calcium, copper, zinc, iron or nickel cation or represents an ammonium cation of the formula 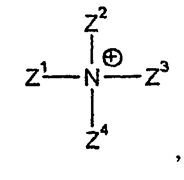 in which
Z¹ represents hydrogen or straight-chain or branched alkyl having 1 to 8 carbon atoms,
Z² represents hydrogen or straight-chain or branched alkyl having 1 to 8 carbon atoms,
Z³ represents hydrogen, straight-chain or branched alkyl having 1 to 8 carbon atoms, benzyl or phenyl and
Z⁴ represents straight-chain or branched alkyl having 1 to 18 carbon atoms, benzyl or phenyl,
and
R¹ represents a radical of the formula 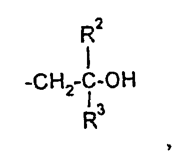 in which
R² represents straight-chain or branched alkyl having 1 to 6 carbon atoms, where these radicals may be mono- to tetrasubstituted by identical or different substituents from the group consisting of halogen, alkoxy having 1 to 4 carbon atoms, alkoximino having 1 to 4 carbon atoms in the alkoxy moiety and cycloalkyl having 3 to 7 carbon atoms,
or
represents straight-chain or branched alkenyl having 2 to 6 carbon atoms, where each of these radicals may be mono- to trisubstituted by identical or different substituents from the group consisting of halogen, alkoxy having 1 to 4 carbon atoms and cycloalkyl having 3 to 7 carbon atoms,
or
represents cycloalkyl having 3 to 7 carbon atoms, where each of these radicals may be mono- to trisubstituted by identical or different substituents from the group consisting of halogen, cyano and alkyl having 1 to 4 carbon atoms,
or
represents aralkyl having 6 to 10 carbon atoms in the aryl moiety and 1 to 4 carbon atoms in the straight-chain or branched alkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2
carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aralkenyl having 6 to 10 carbon atoms in the aryl moiety and 2 to 4 carbon atoms in the alkenyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aroxyalkyl having 6 to 10 carbon atoms in the aryl moiety and 1 to 4 carbon atoms in the straight-chain or branched oxyalkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aryl having 6 to 10 carbon atoms, where each of these radicals may be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents an optionally benzo-fused five- or six-membered heteroaromatic radical having 1 to 3 heteroatoms, such as nitrogen, sulphur and/or oxygen, where each of these radicals may be mono- to trisubstituted by identical or different substituents from the group consisting of halogen, alkyl having 1 to 4 carbon atoms, hydroxyalkyl having 1 to 4 carbon atoms, hydroxyalkynyl having 3 to 8 carbon atoms, alkoxy having 1 or 2 carbon atoms, alkylthio having 1 or 2 carbon atoms, halogenoalkyl, halogenoalkoxy and halogenoalkylthio having in each case 1 or 2 carbon atoms and 1 to 5 identical or different halogen atoms, such as fluorine or chlorine atoms, formyl, dialkoxymethyl having 1 or 2 carbon atoms in each alkoxy group, acyl having 2 to 4 carbon atoms, alkoxycarbonyl having 1 to 4 carbon atoms in the alkoxy moiety, alkoximinoalkyl having 1 to 4 carbon atoms in the alkoxy moiety and 1 to 3 carbon atoms in the alkyl moiety, nitro and cyano, and
R³ represents straight-chain or branched alkyl having 1 to 6 carbon atoms, where these radicals may be mono- to tetrasubstituted by identical or different substituents from the group consisting of halogen, alkoxy having 1 to 4 carbon atoms, alkoximino having 1 to 4 carbon atoms in the alkoxy moiety and cycloalkyl having 3 to 7 carbon atoms,
or
represents straight-chain or branched alkenyl having 2 to 6 carbon atoms, where each of these radicals may be mono- to trisubstituted by identical or different substituents from the group consisting of halogen, alkoxy having 1 to 4 carbon atoms and cycloalkyl having 3 to 7 carbon atoms,
or
represents cycloalkyl having 3 to 7 carbon atoms, where each of these radicals may be mono- to trisubstituted by identical or different substituents from the group consisting of halogen, cyano and alkyl having 1 to 4 carbon atoms,
or
represents aralkyl having 6 to 10 carbon atoms in the aryl moiety and 1 to 4 carbon atoms in the straight-chain or branched alkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aralkenyl having 6 to 10 carbon atoms in the aryl moiety and 2 to 4 carbon atoms in the alkenyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aroxyalkyl having 6 to 10 carbon atoms in the aryl moiety and 1 to 4 carbon atoms in the straight-chain or branched oxyalkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aryl having 6 to 10 carbon atoms, where each of these radicals may be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents an optionally benzo-fused five- or six-membered heteroaromatic radical having 1 to 3 heteroatoms, such as nitrogen, sulphur and/or oxygen, where each of these radicals may be mono- to trisubstituted by identical or different substituents from the group consisting of halogen, alkyl having 1 to 4 carbon atoms, hydroxyalkyl having 1 to 4 carbon atoms, hydroxyalkynyl having 3 to 8 carbon atoms, alkoxy having 1 or 2 carbon atoms, alkylthio having 1 or 2 carbon atoms, halogenoalkyl, halogenoalkoxy and halogenoalkylthio having in each case 1 or 2 carbon atoms and 1 to 5 identical or different halogen atoms, such as fluorine or chlorine atoms, formyl, dialkoxymethyl having 1 or 2 carbon atoms in each alkoxy group, acyl having 2 to 4 carbon atoms, alkoxycarbonyl having 1 to 4 carbon atoms in the alkoxy moiety, alkoximinoalkyl having 1 to 4 carbon atoms in the alkoxy moiety and 1 to 3 carbon atoms in the alkyl moiety, nitro and cyano.

2. Process for preparing triazolyl-mercaptides of the formula (I) according to Claim 1, **characterized in that** mercapto-triazoles of the formula 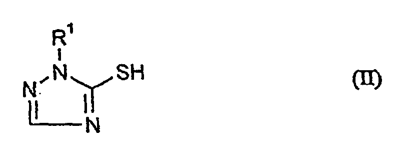 in which
R¹ is as defined above,
are either
a) reacted with hydroxides of the formula
M¹-OH (III)
in which
M¹ represents a sodium or potassium cation, an equivalent of a magnesium or calcium cation or represents an ammonium cation of the formula 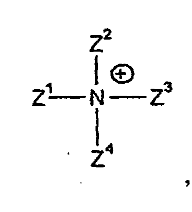
, in which
Z¹, Z², Z³ and Z⁴ are each as defined above, in the presence of a diluent,
or
b) reacted with amines of the formula 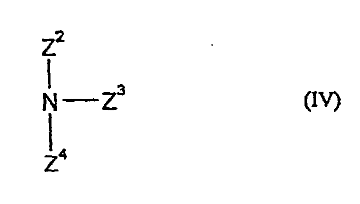 in which
Z², Z³ and Z⁴ are each as defined above, in the presence of a diluent,
or
c) reacted with acetates of the formula
CH₃-COO M² (V)
in which
M² represents an equivalent of a copper, zinc, iron or nickel cation,
in the presence of a diluent.

3. Microbicidal compositions, **characterized in that** they comprise at least one triazolyl-mercaptide of the formula (I) according to Claim 1, in addition to extenders and/or surfactants.

4. The use of triazolyl-mercaptides of the formula (I) according to Claim 1 as microbicides in crop protection and in the protection of materials.

5. A method for controlling undesirable microorganisms in crop protection and in the protection of materials, **characterized in that** triazolyl-mercaptides of the formula (I) according to Claim 1 are applied to the microorganisms and/or their habitat.

6. Process for preparing microbicidal compositions, **characterized in that** triazolyl-mercaptides of the formula (I) according to Claim 1 are mixed with extenders and/or surfactants.

## Revendications

1. Triazolyl-mercaptide de la formule : 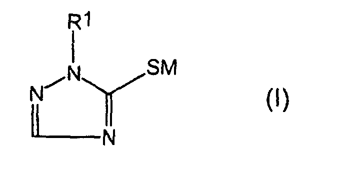 dans laquelle :
M représente un cation sodium ou potassium, un équivalent d'un cation magnésium, calcium, cuivre, zinc, fer ou nickel ou un cation ammonium de la formule : 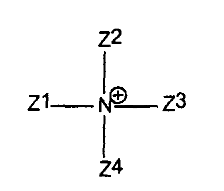
dans laquelle :
Z¹ représente l'atome d'hydrogène ou un radical alcoyle linéaire ou ramifié ayant 1 à 8 atomes de carbone,
Z² représente l'atome d'hydrogène ou un radical alcoyle linéaire ou ramifié ayant 1 à 8 atomes de carbone,
Z³ représente l'atome d'hydrogène ou un radical alcoyle linéaire ou ramifié ayant 1 à 8 atomes de carbone, le radical benzyle ou phényle, et
Z⁴ représente un radical alcoyle linéaire ou ramifié ayant 1 à 18 atomes de carbone, le radical benzyle ou phényle, et
R¹ représente un reste de la formule : 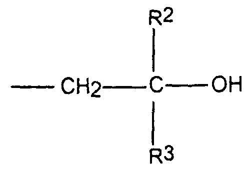
dans laquelle :
R² représente un radical alcoyle linéaire ou ramifié ayant 1 à 6 atomes de carbone, où ces restes peuvent être substitués une à quatre fois, de manière identique ou différente, par un atome d'halogène, un radical alcoxy ayant 1 à 4 atomes de carbone, un radical alcoximino ayant 1 à 4 atomes de carbone dans la partie alcoxy et/ou un radical cycloalcoyle ayant 3 à 7 atomes de carbone, ou
représente un radical alcényle linéaire ou ramifié ayant 2 à 6 atomes de carbone, où chacun de ces restes peut être substitué une à trois fois, de manière identique ou différente, par un atome d'halogène, un radical alcoxy ayant 1 à 4 atomes de carbone et/ou un radical cycloalcoyle ayant 3 à 7 atomes de carbone, ou
représente un radical cycloalcoyle ayant 3 à 7 atomes de carbone, où chacun de ces restes peut être substitué une à trois fois, de manière identique ou différente, par un atome d'halogène, le radical cyano et/ou un radical alcoyle ayant 1 à 4 atomes de carbone, ou
représente un radical aralcoyle ayant 6 à 10 atomes de carbone dans la partie aryle et 1 à 4 atomes de carbone dans la partie alcoyle linéaire ou ramifiée, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aralcényle ayant 6 à 10 atomes de carbone dans la partie aryle et 2 à 4 atomes de carbone dans la partie alcényle, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aroxyalcoyle ayant 6 à 10 atomes de carbone dans la partie aryle et 1 à 4 atomes de carbone dans la partie oxyalcoyle linéaire ou ramifiée, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aryle ayant 6 à 10 atomes de carbone, où chacun de ces restes peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un reste hétéroaromatique ayant cinq à six membres, le cas échéant condensé sur un cycle benzénique, ayant 1 à 3 hétéroatomes comme l'atome d'azote, de soufre et/ou d'oxygène, où chacun de ces restes peut être chaque fois substituée une à trois fois, de manière identique ou différente, par un atome d'halogène, un radical alcoyle ayant 1 à 4 atomes de carbone, un radical hydroxyalcoyle ayant 1 à 4 atomes de carbone, un radical hydroxyalcynyle ayant 3 à 8 atomes de carbone, un radical alcoxy ayant 1 ou 2 atomes de carbone, un radical alcoylthio ayant 1 ou 2 atomes de carbone, des radicaux halogénoalcoyle, halogénoalcoxy et halogénoalcoylthio ayant 1 ou 2 atomes de carbone et 1 à 5 atomes d'halogène identiques ou différents, comme l'atome de fluor ou de chlore, le radical formyle, un radical dialcoxyméthyle ayant 1 ou 2 atomes de carbone dans chaque radical alcoxy, un radical acyle ayant 2 à 4 atomes de carbone, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, et
R³ représente un radical alcoyle linéaire ou ramifié ayant 1 à 6 atomes de carbone, où ces restes peuvent être substitués une à quatre fois, de manière identique ou différente, par un atome d'halogène, un radical alcoxy ayant 1 à 4 atomes de carbone, un radical alcoximino ayant 1 à 4 atomes de carbone dans la partie alcoxy et/ou un radical cycloalcoyle ayant 3 à 7 atomes de carbone, ou
représente un radical alcényle linéaire ou ramifié ayant 2 à 6 atomes de carbone, où chacun de ces restes peut être substitué une à trois fois, de manière identique ou différente, par un atome d'halogène, un radical alcoxy ayant 1 à 4 atomes de carbone et/ou un radical cycloalcoyle ayant 3 à 7 atomes de carbone, ou
représente un radical cycloalcoyle ayant 3 à 7 atomes de carbone, où chacun de ces restes peut être substitué une à trois fois, de manière identique ou différente, par un atome d'halogène, le radical cyano et/ou un radical alcoyle ayant 1 à 4 atomes de carbone, ou
représente un radical aralcoyle ayant 6 à 10 atomes de carbone dans la partie aryle et 1 à 4 atomes de carbone dans la partie alcoyle linéaire ou ramifiée, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aralcényle ayant 6 à 10 atomes de carbone dans la partie aryle et 2 à 4 atomes de carbone dans la partie alcényle, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aroxyalcoyle ayant 6 à 10 atomes de carbone dans la partie aryle et 1 à 4 atomes de carbone dans la partie oxyalcoyle linéaire ou ramifiée, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aryle ayant 6 à 10 atomes de carbone, où chacun de ces restes peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un reste hétéroaromatique ayant cinq à six membres, le cas échéant condensé sur un cycle benzénique, ayant 1 à 3 hétéroatomes comme l'atome d'azote, de soufre et/ou d'oxygène, où chacun de ces restes peut être chaque fois substituée une à trois fois, de manière identique ou différente, par un atome d'halogène, un radical alcoyle ayant 1 à 4 atomes de carbone, un radical hydroxyalcoyle ayant 1 à 4 atomes de carbone, un radical hydroxyalcynyle ayant 3 à 8 atomes de carbone, un radical alcoxy ayant 1 ou 2 atomes de carbone, un radical alcoylthio ayant 1 ou 2 atomes de carbone, des radicaux halogénoalcoyle, halogénoalcoxy et halogénoalcoylthio ayant 1 ou 2 atomes de carbone et 1 à 5 atomes d'halogène identiques ou différents, comme l'atome de fluor ou de chlore, le radical formyle, un radical dialcoxyméthyle ayant 1 ou 2 atomes de carbone dans chaque radical alcoxy, un radical acyle ayant 2 à 4 atomes de carbone, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano.

2. Procédé de préparation de triazolyl-mercaptides de la formule (I) suivant la revendication 1, **caractérisé en ce que** l'on fait réagir un mercaptotriazole de la formule : 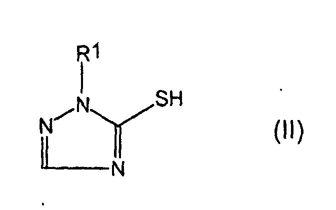 dans laquelle :
R¹ a la signification indiquée ci-dessus,
soit
a) avec des hydroxydes de la formule :
M¹-OH (III)
dans laquelle:
M¹ représente un cation sodium ou potassium, un équivalent d'un cation magnésium ou calcium, ou un cation ammonium de la formule : 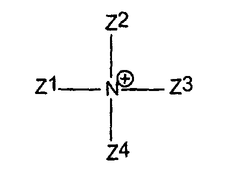
dans laquelle :
Z¹, Z², Z³ et Z⁴ ont les significations données ci-dessus,
en présence d'un agent de dilution,
soit
b) avec des amines de la formule : 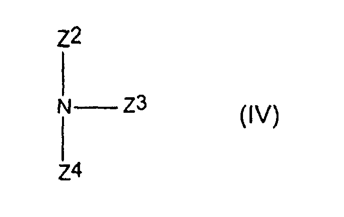 dans laquelle :
Z², Z³ et Z⁴ ont les significations données ci-dessus,
en présence d'un agent de dilution,
soit
c) avec des acétates de la formule :
CH₃-COO M² (V)
dans laquelle:
M² représente un équivalent d'un cation cuivre, zinc, fer ou nickel,
en présence d'un agent de dilution.

3. Agent microbicide, **caractérisé par** une teneur en au moins un triazolyl-mercaptide de la formule (I) suivant la revendication 1, avec un agent de dilution et/ou un tensioactif.

4. Utilisation de triazolyl-mercaptides de la formule (I) suivant la revendication 1, comme microbicide dans la protection des végétaux et dans la protection des matériaux.

5. Procédé pour lutter contre les microorganismes non souhaités dans la protection des végétaux et dans la protection des matériaux, **caractérisé en ce que** l'on applique des triazolyl-mercaptides de la formule (I) suivant la revendication 1, sur les microorganismes et/ou leur biotope.

6. Procédé de préparation d'agents microbicides, **caractérisé en ce que** l'on mélange un triazolyl-mercaptide de la formule (I) suivant la revendication 1, avec un agent de dilution et/ou un tensioactif.
